# EUROPÄISCHE PATENTSCHRIFT

(11) **EP 1 986 959 B1**
(45) Veröffentlichungstag und Bekanntmachung des Hinweises auf die Patenterteilung: **27.10.2010**
(21) Anmeldenummer: 07703453.6
(22) Anmeldetag: 14.02.2007
(51) Int. Cl.: C02F 1/46, C02F 1/467, C25B 1/26, C02F 1/461

(54) **VERFAHREN ZUR HERSTELLUNG EINES DESINFEKTIONSMITTELS DURCH ELEKTROCHEMISCHE AKTIVIERUNG (ECA) VON WASSER**
PROCESS FOR PRODUCING A DISINFECTANT BY ELECTROCHEMICAL ACTIVATION (ECA) OF WATER
PROCÉDÉ DE PRODUCTION D'UN DÉSINFECTANT PAR ACTIVATION ÉLECTROCHIMIQUE DE L'EAU

(30) Priorität: 17.02.2006 DE 102006007931; 11.09.2006 DE 102006043267
(43) Veröffentlichungstag der Anmeldung: 05.11.2008
(73) Patentinhaber: Actides Gmbh, 69518 Abtsteinach (DE)
(72) Erfinder: SALATHE, Peter, 4410 Liestal (CH); FISCHER, Christian, 69483 Wald-Michelbach (DE); JÖST, Bernd, 69518 Abtsteinach (DE); GROSS, Steven, 64743 Beerfelden (DE); SCHMIDT, Volkmar, 68519 Viernheim (DE)
(74) Vertreter: Lenz, Steffen
(86) Internationale Anmeldenummer: PCT/EP2007/001265
(87) Internationale Veröffentlichungsnummer: WO 2007/093395

(56) Entgegenhaltungen:
- EP-A- 0 737 482
- EP-A- 1 382 573
- EP-A1- 0 612 694
- EP-A1- 0 645 141
- WO-A-00/33757
- US-A- 5 427 667
- US-A- 6 126 796
- US-A1- 2003 164 286
- US-A1- 2003 185 704
- US-B1- 6 655 394
- US-B2- 6 589 396

## Beschreibung

Die Erfindung betrifft ein Verfahren zur Herstellung eines Desinfektionsmittels durch elektrochemische Aktivierung (ECA) von Wasser, indem dem Wasser eine Elektrolytlösung, insbesondere eine Natrium- und/oder Kaliumchloridlösung, zugesetzt und das mit der Elektrolytlösung beaufschlagte Wasser in Form einer verdünnten Wasser-/Elektrolytlösung in einem Elektrolysereaktor mit wenigstens einem Kathodenraum mit einer Kathode und mit wenigstens einem von dem Kathodenraum räumlich, insbesondere mittels eines Diaphragmas oder einer Membran, getrennten Anodenraum mit einer Anode durch Anlegen einer Gleichspannung an die Elektroden mit einem elektrischen Strom beaufschlagt wird, um die verdünnte Wasser-/Elektrolytlösung in einen zur Desinfektion geeigneten, metastabilen Zustand zu versetzen.

Das Verfahren der elektrochemischen Aktivierung bzw. Behandlung ist insbesondere zur Desinfektion von Wasser bekannt. Hierbei wird eine verdünnte Lösung eines Elektrolyts, insbesondere eines Neutralsalzes, wie Natriumchlorid (NaCl) bzw. Kochsalz, Kaliumchlorid (KCl) oder dergleichen, in einem Elektrolysereaktor durch Anlegen einer Spannung an dessen Elektroden in einen zur Desinfektion geeigneeten, aktiven Zustand überführt, welcher in aller Regel metastabiler Natur ist und je nach Art des Wassers und den eingestellten Verfahrensparametern über längere Zeit andauern kann. Der Elektrolysereaktor weist einen Kathodenraum mit einer oder mehreren Kathoden sowie einen Anodenraum mit einer oder mehreren Anoden auf, wobei der Anodenraum und der Kathodenraum mittels eines elektrisch leitfähigen - insbesondere eines für Ionen leitfähigen - Diaphragmas bzw. mittels einer Membran mit den genannten Eigenschaften räumlich voneinander getrennt sind, um eine Vermischung der in beiden Räumen befindlichen Wasser-/Elektrolytlösung zu verhindern. Während bei der Elektrolyse in der Regel ein im wesentlichen vollständiger Umsatz der eingesetzten Edukte - im Falle des Einsatzes einer Natriumchloridlösung zu Chlorgas (Cl₂) und Natronlauge (NaOH), im Falle des Einsatzes einer Kaliumchloridlösung zu Chlorgas und Kalilauge (KOH) - unter Einsatz hoch konzentrierter Elektrolytlösungen angestrebt wird, um vornehmlich die Chlorgasausbeute zu maximieren, wird die Wasser-/Elektrolytlösung bei der elektrochemischen Aktivierung demgegenüber in erheblich verdünnterer Form, in der Regel in einer Konzentration von maximal 20 g/1, vorzugsweise maximal 10 g/1, dem Elektrolysereaktor aufgegeben und lediglich zu einem sehr geringen Anteil umgesetzt, um die physikalischen und chemischen Eigenschaften der Lösung in vorteilhafter Weise zu verändern und insbesondere das Redoxpotential des mit dem Elektrolyt versetzten Wassers zu erhöhen, wodurch eine desinfizierende Wirkung erhalten wird. Entsprechend werden die Reaktionsbedingungen, wie Druck, Temperatur, Elektrodenstrom etc., bei der elektrochemischen Aktivierung im allgemeinen moderater gewählt als bei der Chlor-Alkalielektrolyse. Von Vorteil bei einer solchen elektrochemischen Behandlung, welche im Rahmen der vorliegenden Anmeldung als "elektrochemische Aktivierung" bezeichnet ist, ist insbesondere die gute Gesundheits- und Umweltverträglichkeit der anläßlich der elektrochemischen Aktivierung erzeugten Stoffe in deren jeweiligen Konzentrationen, welche gemäß der deutschen Trinkwasserverordnung (TrinkwV) auch zugelassen sind.

Wie bei der Elektrolyse findet auch bei der elektrochemischen Aktivierung an der Anode (d.h. an der positiv geladenen Elektrode) eine Oxidation statt, während an der Kathode (d.h. an der negativ geladenen Elektrode) eine Reduktion stattfindet. Beim Einsatz einer verdünnten Neutralsalzlösung, wie einer Natriumchloridlösung, wird an der Kathode gemäß der nachfolgenden Reaktionsgleichung (1) vornehmlich Wasserstoff erzeugt:

2 H₂O + 2 e⁻ ---> H₂ + 2 OH⁻ (1),

welcher nach Ausgasen aus der Lösung z.B. aus dem Kathodenraum des Reaktors abgeführt wird. Darüber hinaus wird die verdünnte Wasser-/Elektrolytlösung in dem Kathodenraum des Elektrolysereaktors durch die Bildung von Hydroxidionen alkalisch.

An der Anode werden gemäß den nachfolgenden Reaktionsgleichungen (2) und (3) insbesondere die chemischen Oxidationsmittel Sauerstoff (O₂) und Chlor (Cl₂) erzeugt, welche bekanntermaßen hinsichtlich einer Desinfektion von Wasser wirksam sind. Ferner ist zu beachten, daß infolge der Bildung von H₃O⁺-Ionen die verdünnte Wasser-/Elektrolytlösung in dem Anodenraum des Elektrolysereaktors sauer wird:

6 H₂O ---> O₂ + 4 H₃O⁺ + 4 e⁻ (2),

2 Cl⁻ ---> Cl₂ + 2 e⁻ (3).

Chlor wiederum dissoziiert in Wasser entsprechend der nachfolgenden Gleichgewichtsreaktion (4) in Hypochloritionen (OCl⁻) und Chloridionen (Cl⁻), welche sich wiederum mit einem geeigneten Kation, z.B. Na⁺ aus dem Elektrolyt, oder mit einem Proton bzw. einem H₃O⁺-Ion zu dem entsprechenden (Natrium)salz bzw. zu der entsprechenden Säure, d.h. zu hypochloriger Säure (HClO) und Chlorwasserstoff bzw. verdünnter Salzsäure (HCl) reagieren können:

Cl₂ + 3 H₂O <===> 2 H₃O⁺ + OCl⁻ + Cl⁻ (4).

Ferner können aus den vorgenannten, an der Anode gebildeten Stoffen durch Sekundärreaktionen weitere Stoffe erzeugt werden, welche ebenfalls bekanntermaßen im Hinblick auf eine Desinfektion von Wasser wirksam sind. Hierbei handelt es sich insbesondere um Wasserstoffperoxid (H₂O₂, Reaktionsgleichung (5)), Ozon (O₃, Reaktionsgleichung (6)), Chlordioxid (ClO₂, Reaktionsgleichung (7)), Chlorate (ClO₃⁻, Reaktionsgleichung (8)) und verschiedene Radikale (Reaktionsgleichungen (9) und (10)).

4 H₂O ---> H₂O₂ + 2 H₃O⁺ + 2 e⁻ (5),

O₂ + 3 H₂O ---> O₃ + 2 H₃O⁺ + 2 e⁻ (6),

Cl⁻ + 4 OH⁻ ---> ClO₂ + 2 H₂O + 5 e⁻ (7),

3 OCl⁻ ---> ClO₃⁻ + 2 Cl⁻ (8),

5 H₂O ---> HO₂ + 3 H₃O⁺ + 3 e⁻ (9),

H₂O₂ + H₂O ---> HO₂ + H₃O⁺ + e⁻ (10).

Nachteilig bei dem Verfahren der elektrochemischen Aktivierung ist insbesondere die mangelnde Qualitätskontrolle, da die für eine hinreichende Desinfektion des Wassers erforderlichen, meist empirisch ermittelten Verfahrensparameter, wie Menge der zugesetzten Elektrolytlösung, eingestellter Elektrodenspannung bzw. -strom und dergleichen, nicht nur von dem eingesetzten Elektrolysereaktor, wie dessen Reaktionsvolumen, dessen Anoden- und Kathodenfläche, der Verweilzeit des zu desinfizierenden Wassers in dem Reaktor etc., sondern insbesondere auch von der Zusammensetzung des jeweiligen, zu desinfizierenden Wassers, wie insbesondere dessen Leitfähigkeit und dessen Redoxpotential, abhängen. Dabei können für ein bestimmtes Wasser - zumeist empirisch - ermittelte Verfahrensparameter, welche bei diesem Wasser zu einer zufriedenstellenden Desinfektionswirkung führen, bei einem anderen Wasser zu einer nur sehr mangelhaften Desinfektionswirkung führen.

Ferner hat sich insbesondere gezeigt, daß die mittels elektrochemischer Aktivierung gemäß dem Stand der Technik hergestellten Lösungen in der Regel - teils hochgradig - mit unerwünschten Produkten verunreinigt sind, wobei häufig gemäß der obigen Reaktionsgleichung (3) praktisch ausschließlich Chlorgas (Cl₂) erzeugt wird, welches bei herkömmlichen Elektrolyseprozessen zwar erwünscht, bei der elektrochemischen Aktivierung zur Erzeugung eines Desinfektionsmittels jedoch gerade nicht erwünscht ist, da es einen stechenden Geruch der elektrochemisch aktivierten Lösung verursacht. Überdies lassen sich aufgrund der in Abhängigkeit der vorgenannten Parameter stark schwankenden Zusammensetzung der elektrochemisch aktivierten Lösung keine zuverlässigen Angaben hinsichtlich der Haltbarkeit bzw. Lagerfähigkeit der elektrochemisch aktivierten Lösung treffen, so daß praktisch nur eine Produktion derselben vor Ort in Betracht kommt. In der Praxis hat sich das Verfahren daher - vornehmlich aufgrund seiner schlechten Handhabbarkeit bzw. der nur unzureichend möglichen Garantie einer hinreichenden Desinfektion - am Markt nicht durchsetzen können.

Ein Verfahren zur Erzeugung eines Desinfektionsmittels mittels elektrochemischer Aktivierung ist beispielsweise aus der DE 20 2005 008 695 U1 bekannt.

Die US 5 427 667 A beschreibt einen Elektrolysereaktor zur elektrochemischen Aktivierung von Wasser, welcher unter anderem zur Aufreinigung von Trinkwasser vorgesehen sein kann. In der Druckschrift sind Ausführungsbeispiele von solchermaßen an der Anode erzeugten Desinfektionsmitteln offenbart, welche einerseits einen pH-Wert von 3,5 und ein Redoxpotential von 1000 mV bzw. 1100 mV (bezogen auf die Silber-/Silberchlorid-Referenzelektrode), andererseits einen pH-Wert von 2,7 und ein Redoxpotential von 1150 mV (ebenfalls bezogen auf die Ag/AgCl-Elektrode) aufweisen. Eine Steuerung des Verfahrens auf einen solchen pH- und/oder Redoxpotentialwertebereich ist indes nicht vorgesehen.

Der Erfindung liegt daher die Aufgabe zugrunde, ein Verfahren zur Herstellung eines Desinfektionsmittels durch elektrochemische Aktivierung (ECA) von Wasser der eingangs genannten Art dahingehend weiterzubilden, daß eine im wesentlichen gleichbleibend hohe Desinfektionswirkung des Desinfektionsmittels bzw. des zu desinfizierenden Wassers sichergestellt ist, welche insbesondere auch der deutschen Trinkwasserverordnung genügt.

Diese Aufgabe wird erfindungsgemäß bei einem Verfahren der eingangs genannten Art dadurch gelöst, daß der pH-Wert der verdünnten Wasser-/Elektrolytlösung in dem Anodenraum auf einen Wert zwischen 2,7 und 3,5 und das Redoxpotential der verdünnten Wasser-/Elektrolytlösung in dem Anodenraum auf einen Wert zwischen 1240 mV und 1360 mV bezogen auf die Standardwasserstoffelektrode (SHE) gesteuert wird, indem
(a) bei einer vorgegebenen Verweilzeit (Tᵥ) der Wasser-/Elektrolytlösung in dem Elektrolysereaktor und/oder bei einem vorgegebenen Volumenstrom (V') der Wasser-/Elektrolytlösung durch den Elektrolysereaktor ein von der jeweiligen Zusammensetzung des zu desinfizierenden Wassers abhängiger, statischer Sollstrom (I_{soll, stat}) zwischen den Elektroden in Abhängigkeit von der Leitfähigkeit (κ) und/oder von der Härte (H) des Wassers gemäß einer Geradengleichung der Form

   I_{soll, stat} = K₁ κ + K₂

   und/oder

   I_{soll, stat} = K3 · H + K₄

   ermittelt wird, wobei K₁, K₂ , K₃ und K₄ reaktorspezifische Konstanten sind; und
(b) bei einem vorgegebenen statischen Sollstrom (I_{soll, stat}) zwischen den Elektroden des Elektrolysereaktors ein von der jeweiligen Verweilzeit (Tᵥ) der Wasser-/Elektrolytlösung in dem Elektrolysereaktor und/ oder von dem jeweiligen Volumenstrom der Wasser-/Elektrolytlösung durch den Elektrolysereaktor abhängiger, dynamischer Sollstrom (I_{soll, dyn}) gemäß einer Geradengleichung der Form

   I_{soll, dyn} = K₅ · Tᵥ + K₆

   und/oder

   I_{soll, dyn} = K₇ · V' + K₈

   ermittelt wird, wobei K₅, K₆, K₇ und K₈ reaktorspezifische Konstanten sind; und
(c) an die Elektroden des Elektrolysereaktors ein Gesamt-Sollstrom (T_{soll, ges}) angelegt wird, welcher aus der Summe des statischen Sollstromes (I_{soll, stat}) und des dynamischen Sollstromes (I_{soll, dyn}) gebildet ist:

   I_{soll, ges} = I_{soll, stat} + I_{soll, dyn}.

Überraschenderweise wurde gefunden, daß sich bei einer Einstellung des pH-Wertes der verdünnten Wasser-/Elektrolytlösung, d.h. des zu desinfizierenden Wassers mit dem in dieses eingemischten Elektrolyt, wie der Natrium- oder Kaliumchloridlösung, in dem Anodenraum des Elektrolysereaktors auf einen Wert zwischen etwa 2,7 und etwa 3,5, vorzugsweise auf einen Wert zwischen etwa 2,7 und etwa 3,3, insbesondere auf einen Wert zwischen etwa 2,8 und etwa 3,2, z.B. auf einen Wert zwischen etwa 2,9 und etwa 3,1, wie auf einen Wert von etwa 3,0, nicht nur eine praktisch gleichbleibende Desinfektionswirkung für Trink- und Brauchwässer mit praktisch beliebiger Zusammensetzung, sondern auch eine hinreichende Depotwirkung ergibt, welche keine weiteren Desinfektionsschritte mehr erforderlich macht und welche insbesondere auch im Falle von Stoßbelastungen anhält. Die erfindungsgemäße Steuerung des pH-Wertes der verdünnten Wasser-/Elektrolytlösung in dem Anodenraum des Elektrolysereaktors führt zu einer potentialkontrollierten anodischen Oxidation (PAO), wobei sich ein Redoxpotential zwischen etwa 1240 mV und etwa 1360 mV bezogen auf die Standardwasserstoffelektrode (SHE), vorzugsweise zwischen etwa 1280 mV und etwa 1360 mV bezogen auf die SHE, insbesondere zwischen etwa 1320 mV und etwa 1360 mV bezogen auf die SHE, einstellt. Untersuchungen haben nicht nur gezeigt, daß sich die als Testkeime eingesetzten *Escherichia coli, Pseudomonas aeruginosa* und *Enterococcus faecium* bei einer Verdünnung der auf die erfindungsgemäße Weise durch elektrochemische Aktivierung unter potentialkontrollierter anodischer Oxidation hergestellten, anodischen, verdünnten Wasser-/Elektrolytlösung von etwa 1:400 innerhalb von etwa 30 s um mehr als vier Zehnerpotenzen reduzieren lassen, sondern daß auch ältere Rohrleitungen mit einem bereits optisch sichtbaren Befall von einem Biofilm oder Biorasen innerhalb von zwei bis vier Wochen praktisch vollständig von dem Biorasen befreit werden. Folglich stellt bereits eine Verdünnung von etwa 1:400 eine z.B. zur Desinfektion von Trink- und Brauchwasser oder Schwimmbadwasser geeignete, höchst effiziente Verdünnung dar. Wie weiter unten noch näher erläutert, ist es natürlich auch möglich, die auf die erfindungsgemäße Weise elektrochemisch aktivierte, anodische, verdünnte Wasser-/Elektrolytlösung - z.B. in konzentrierterer Form - für eine Vielzahl an weiteren Anwendungszwecken einzusetzen. Ferner ist es selbstverständlich auch möglich, im Falle einer Desinfektion von Wasser, z.B. auch nur über einen begrenzten Zeitraum, höhere Konzentrationen bzw. geringere Verdünnungen der anodischen Wasser-/Elektrolytlösung dem zu desinfizierenden Wasser zuzusetzen, um im Falle von in Rohrleitungssystemen aufgetretenen Kalamitäten für eine wirksame Akutbehandlung zu sorgen. Solche Verdünnungen können dann z.B. zwischen etwa 1:100 bis etwa 1:200 betragen, wobei die Verdünnung natürlich grundsätzlich von dem jeweiligen Anwendungsfall abhängt.

Darüber hinaus wurde gefunden, daß die Einstellung des pH-Wertes des Desinfektionsmittels in Form der anodischen, verdünnten Wasser-/Elektrolytlösung in dem Elektrolysereaktor auf den erfindungsgemäßen pH-Wertbereich keine dauerhafte Absenkung des pH-Wertes des zu desinfizierenden Wassers auf diesen Wert bewirkt, was aufgrund des verhältnismäßig sauren pH-Wertes für viele potentielle Anwendungsmöglichkeiten der elektrochemischen Aktivierung, z.B. für Trinkwässer, unerwünscht sein könnte. Dies liegt einerseits an der nur sehr geringen, zur Desinfektion des Wassers erforderlichen zudosierten Menge des Desinfektionsmittels (z.B. in einer Verdünnung von etwa 1:300 bis 1:500), welche je nach Pufferkapazität des Wassers auch bei sehr weichen Wässern eine pH-Wertabsenkung von höchstens etwa -0,2 verursacht. Andererseits steigt der pH-Wert nach einiger Zeit wieder an, wobei vermutet wird, daß dies auf den Zerfall der anläßlich der elektrochemischen Aktivierung erzeugten, metastabilen Stoffe, wie Ozon, verschiedenen Radikalen etc. (vgl. auch die obigen Reaktionsgleichungen), zurückzuführen ist. Gleichwohl wird jedoch - wie bereits erwähnt - die hervorragende Desinfektionswirkung erreicht.

Überdies läßt sich überraschenderweise die Entstehung von Chlorgas gemäß der obigen Reaktionsgleichung (3) auf ein Minimum begrenzen, so daß das Desinfektionsmittel in Form der elektrochemisch aktivierten, anodischen, verdünnten Wasser-/Elektrolytlösung allenfalls einen sehr schwachen Chlorgeruch besitzt, während das desinfizierte Produkt, wie Wasser, welchem diese Lösung in der geeigneten Verdünnung zugesetzt worden ist, überhaupt keinen für Chlor typischen Geruch aufweist. Infolge der potentialkontrollierten anodischen Oxidation werden vornehmlich Hypochlorite, wie Natriumhypochlorit (NaClO) und hypochlorige Säure (HOCl), metastabile radikalische Verbindungen sowie in geringeren Mengen Chlorwasserstoff anstelle von Chlorgas (Cl₂) erzeugt, d.h. das Gleichgewicht der obigen Reaktionsgleichung (4) wird durch die erfindungsgemäße Verfahrensführung offenbar nach rechts verschoben.

Aufgrund der räumlichen Trennung des Anodenraums, in welchem die zu Desinfektionszwecken hoch wirksame, elektrochemisch aktivierte, verdünnte Wasser-/Elektrolytlösung erzeugt wird, von dem Kathodenraum wird eine Vermischung der anläßlich der elektrochemischen Aktivierung im Anodenraum erzeugten Produkte mit den im Kathodenraum erzeugten Produkten und somit die Bildung von zur Desinfektion des Wassers wenig(er) geeigneten Stoffen zu verhindern. Dies kann z.B. durch eine Trennung des Anodenraumes von dem Kathodenraum des Elektrolysereaktors mittels eines Diaphragmas, einer Membran oder dergleichen geschehen, welche(s) elektrisch leitfähig, aber im wesentlichen flüssigkeitsdicht ausgebildet ist. In diesem Zusammenhang hat sich beispielsweise ein(e) Diaphragma/Membran aus porösem Zirkoniumdioxid (ZrO₂) und/oder aus porösem Aluminiumoxid (Al₂O₃) als geeignet erwiesen.

Mit "Steuerung des pH-Wertes auf einen Wert im Bereich von 2,7 bis 3,5" ist im Sinne der Erfindung im übrigen gemeint, daß die aus dem Anodenraum des Elektrolysereaktors, welcher von dem Kathodenraum durch ein(e) elektrisch leitfähige(s) Diaphragma/Membran räumlich getrennt ist, austretende Lösung - bzw, die elektrochemisch aktivierte, anodische, verdünnte Wasser-/Elektrolytlösung -, einen solchen pH-Wert aufweist, d.h. die Steuerung des pH-Wertes geschieht derart, daß sich dieser pH-Wert am Ende des Reaktors in dessen Anodenraum eingestellt hat. Entsprechendes gilt für die "Steuerung des Redoxpotentials der verdünnten Wasser-/Elektrolytlösung" auf einen Wert zwischen 1240 mV und 1360 mV bezogen auf die SHE, welche derart zu geschehen hat, daß die aus dem Anodenraum des Elektrolysereaktors austretende Lösung - bzw. die elektrochemisch aktivierte, anodische, verdünnte Wasser-/Elektrolytlösung - ein solches Redoxpotential besitzt, d.h. die Steuerung des Redoxpotentials geschieht derart, daß sich dieser Wert am Ende des Reaktors in dessen Anodenraum eingestellt hat. Wie bereits erwähnt, bezieht sich das Redoxpotential stets auf die Normal- (NHE) bzw. Standardwasserstoffelektrode (SHE). Mit "Steuerung" ist sowohl eine geeignete, mehr oder minder statische Voreinstellung der jeweiligen Verfahrensparameter als auch eine dynamische Regelung der Verfahrensparameter während des Reaktorbetriebs angesprochen, wie sie nachfolgend näher erläutert ist.

Wie aus den obigen, an der Anode stattfindenden Reaktionsgleichungen ersichtlich, werden bei den an der Anode ablaufenden Oxidationsreaktionen vielfach Protonen bzw. H₃O⁺-Ionen erzeugt, welche den pH-Wert folglich herabsetzen. Aus diesem Grund ist es möglich, den pH-Wert allein durch die an der Anode ablaufenden Reaktionen innerhalb des erfindungsgemäßen Bereiches einzustellen, wobei eine Erhöhung des bei der elektrochemischen Aktivierung erzielten Umsatzes an der Anode eine vermehrte Erzeugung von Protonen und folglich einen geringeren pH-Wert ergibt. Der anläßlich der elektrochemischen Aktivierung erhaltene Umsatz kann bei einer vorgegebenen Reaktorgeometrie wiederum dadurch erhöht werden, indem ein erhöhter Stromfluß zwischen den Elektroden eingestellt bzw. indem an die Elektroden eine höhere Spannung angelegt wird (welche einen höheren elektrischen Stromfluß zwischen den Elektroden zur Folge hat), indem die Verweilzeit der verdünnten Wasser-/Elektrolytlösung in dem Reaktor erhöht wird (bzw. - sofern eine kontinuierliche oder semikontinuierliche Verfahrensführung vorgesehen ist - indem der Volumenstrom der verdünnten Wasser-/Elektrolytlösung durch den Reaktor verlangsamt wird), und/oder indem mehr Elektrolytlösung, z.B. mehr Natrium-/Kaliumchloridlösung, zudosiert wird, also eine erhöhte Eduktmenge eingesetzt wird. Gleichwohl sollte, wie eingangs bereits erwähnt, die Gesamtkonzentration an Natrium-/Kaliumchlorid etwa 20 g/l jedenfalls nicht übersteigen.

Andererseits ist es aufgrund dieser Abhängigkeit des pH-Wertes der verdünnten Wasser-/Elektrolytlösung von dem Umsatz des Elektrolyts in dem Anodenraum des Reaktor auch möglich, den pH-Wert unmittelbar oder mittelbar zu messen, um auf diese Weise einen Ist-Wert zu erhalten, welcher - z.B. mittels herkömmlicher steuerungstechnischer Einrichtungen, wie mittels eines PID-Reglers - auf den erfindungsgemäßen Soll-Wert gesteuert werden kann. Der pH-Wert der verdünnten Wasser-/Elektrolytlösung kann z.B. unmittelbar mittels eines pH-Meters gemessen werden. Alternativ oder zusätzlich ist es möglich, daß der pH-Wert der verdünnten Wasser-/Elektrolytlösung mittelbar über den zwischen den Elektroden fließenden Strom gemessen wird (z.B. über die zur Erzielung eines solchen Stroms erforderliche Spannung, welche an die Elektroden angelegt werden muß) und/oder daß der pH-Wert der verdünnten Wasser-/Elektrolytlösung mittelbar über die Verweilzeit in dem bzw. - im Falle einer (semi)kontinuierlichen Verfahrensführung - über den Volumenstrom derselben durch den Elektrolysereaktor gemessen wird.

Erfindungsgemäß wird der pH-Wert der verdünnten Wasser-/Elektrolytlösung in dem Anodenraum durch Steuerung des zwischen den Elektroden fließenden Stromes gesteuert, indem z.B. an die Elektroden des Elektrolysereaktors eine geeignete Spannung angelegt wird, welche für einen zur Erzielung des erfindungsgemäßen pH-Wertebereiches erforderlichen Stromfluß sorgt.

Zur erfindungsgemäßen Steuerung des pH-Wertes der verdünnten Wasser-/Elektrolytlösung in dem Anodenraum des Elektrolysereaktors wird dabei bei einer vorgegebenen Verweilzeit (Tᵥ) der Wasser-/Elektrolytlösung in dem Elektrolysereaktor und/oder bei einem vorgegebenen Volumenstrom (V') der Wasser-/Elektrolytlösung durch den Elektrolysereaktor ein von der jeweiligen Zusammensetzung des zu desinfizierenden Wassers abhängiger, statischer Sollstrom (I_{soll, stat}) zwischen den Elektroden ermittelt, wobei dieser statische Sollstrom (I_{soll, stat}) in Abhängigkeit von der Leitfähigkeit (κ) und/oder von der Härte (H) des Wassers ermittelt wird. Der statische Sollstrom I_{soll, stat} wird hierbei gemäß einer Geradengleichung der Form

I_{soll, stat} = K₁ · κ + K₂

und/oder

I_{soll, stat} = K₃ · H + K₄

ermittelt, wobei K₁, K₂, K₃ und K₄ reaktorspezifische Konstanten sind.

Um zusätzlich zu einer mehr oder minder statischen Steuerung des anodischen Potentials des Elektrolysereaktors der oben genannten Art für eine dynamische Regelung in Abhängigkeit der gegebenenfalls sich über die Zeit verändernden, tatsächlichen Parameter zu sorgen, ist erfindungsgemäß darüber hinaus vorgesehen, daß zur Steuerung des pH-Wertes der verdünnten Wasser-/Elektrolytlösung in dem Anodenraum des Elektrolysereaktors bei einem vorgegebenen statischen Sollstrom (I_{soll, stat}) zwischen den Elektroden des Elektrolysereaktors ein von der jeweiligen Verweilzeit der Wasser-/Elektrolytlösung in dem Elektrolysereaktor und/oder von dem jeweiligen Volumenstrom der Wasser-/Elektrolytlösung durch den Elektrolysereaktor abhängiger, dynamischer Sollstrom (I_{soll, dyn}) ermittelt wird, so daß die Steuerung des Reaktors zum Zwecke einer Aufrechterhaltung des pH-Wertes der elektrochemisch aktivierten, anodischen Wasser-/Elektrolytlösung im Bereich von 3 in situ in Abhängigkeit der gemessenen tatsächlichen Parameter, wie der Verweilzeit bzw. des Volumenstroms, geschieht. Dieser dynamische Sollstrom (I_{soll, dyn}) wird hierbei ebenfalls gemäß einer Geradengleichung der Form

I_{soll, dyn} = K₅ · Tᵥ + K₆

und/oder

I_{soll, dyn} = K₇ · V' + K₈

ermittelt, wobei K₅, K₆, K₇ und K₈ reaktorspezifische Konstanten sind. An die Elektroden des Elektrolysereaktors wird bei der erfindungsgemäßen Steuerung, welche sowohl statische, von dem jeweils eingesetzten Wasser abhängige Einstellkomponenten als auch dynamische, von den tatsächlich gemessenen Parametern abhängige Regelungskomponenten umfaßt, dann ein Gesamt-Sollstrom (I_{soll, ges}) angelegt, welcher aus der Summe des statischen Sollstromes (I_{soll, stat}) und des dynamischen Sollstromes (I_{soll, dyn}) gebildet ist:

I_{soll, ges} = I_{soll, stat} + I_{soll, dyn}.

Ein besonderer Vorteil der dynamischen Regelungskomponente der erfindungsgemäßen Steuerung besteht darin, daß in dem Fall, wenn die gemessene Verweilzeit der verdünnten Wasser-/Elektrolytlösung und/oder der gemessene Volumenstrom derselben in dem bzw. durch den Elektrolysereaktor absinkt, bei einem solchen gemessenen Abfall dieser Verweilzeit und/oder dieses Völumenstroms der zwischen den Elektroden fließende Gesamtstrom (I_{soll, ges}) kurzzeitig aufgrund des dynamischen Anteils (I_{soll, dyn}) ebenfalls abgesenkt werden kann. Es wurde festgestellt, daß sich insbesondere im Falle eines kontinuierlichen Betriebs der elektrochemischen Aktivierung Gasbläschen bilden können, wie insbesondere aus dem zu desinfizierenden Wasser ausgasender Sauerstoff sowie Chlorgas, wodurch der bei der elektrochemischen Aktivierung erzielte Umsatz beeinträchtigt wird und folglich auch der pH-Wert ansteigt und wobei ferner eine Verminderung des Volumenstroms durch den Reaktor detektierbar ist. Um dem entgegenzuwirken, hat sich insbesondere eine kurzzeitige Erniedrigung des zwischen den Elektroden des Elektrolysereaktors fließenden Stromes als vorteilhaft erwiesen, was durch den dynamischen Anteil des Sollstroms möglich ist. Eine solche Maßnahme der dynamischen Steuerung bzw. Regelung kann ferner auch unabhängig von der erfindungsgemäß vorgesehenen statischen Steuerung des pH-Wertes des Wasser-/Elektrolytlösung in Abhängigkeit von der Leitfähigkeit bzw. von der Härte des Wassers zweckmäßig sein, um eine Bildung von Gasbläschen in dem Elektrolysereaktor, welche offenbar auf eine Art labiles Gleichgewicht zurückzuführen ist, zu beseitigen und wieder zu einem für eine einwandfreie Desinfektion des Wassers geeigneten Betrieb der elektrochemischen Aktivierung zurückzukehren.

Darüber hinaus kann vorgesehen sein, daß der pH-Wert der verdünnten Wasser-/Elektrolytlösung in dem Anodenraum des Elektrolysereaktors durch gesteuertes Zusetzen der entsprechenden Menge an Elektrolytlösung gesteuert wird, d.h. es wird mehr Elektrolytlösung, d.h. mehr Edukt, zugesetzt, wenn der pH-Wert erniedrigt werden muß, um in den erfindungsgemäßen Bereich gesteuert zu werden, während weniger Elektrolytlösung, d.h. weniger Edukt; zugesetzt wird, wenn der pH-Wert erhöht werden muß, um in den erfindungsgemäßen Bereich gesteuert zu werden.

Die Zudosierung der entsprechenden Menge an Elektrolytlösung, z.B. Natrium-/Kaliumchloridlösung, kann beispielsweise mittels einer Dosierpumpe geschehen, wobei die hierdurch erzeugte Wasser-/Elektrolytlösung vor dem Eintritt in den Elektrolysereaktor vorzugsweise möglichst homogen vermischt wird, um für einen gleichmäßigen Betrieb des Elektrolysereaktors zu sorgen. Als geeignete Mischeinrichtung hat sich insbesondere ein Kugelmischer bewährt, bei welchem die verdünnte Wasser-/Elektrolytlösung durch eine Kugelschüttung geleitet wird.

Ferner ist es zusätzlich möglich, daß der pH-Wert der verdünnten Wasser-/Elektrolytlösung in dem Anodenraum durch Steuerung der Verweilzeit und/oder des Volumenstroms derselben in dem bzw. durch den Elektrolysereaktor gesteuert wird. In jedem Fall können ein oder mehrere Parameter im wesentlichen auf einem konstanten Wert gehalten bzw. geregelt werden.

Um einen Elektrolysereaktor mit einer vorgegebenen Reaktorgeometrie in Abhängigkeit des zu desinfizierenden Wassers zu kalibrieren, d.h. um eine Einstellung der für eine Steuerung des pH-Wertes des verdünnten Wasser-/Elektrolytlösung auf den erfindungsgemäßen Wertebereich erforderlichen Verfahrensparameter vorzunehmen, sieht eine vorteilhafte Ausgestaltung des Verfahrens vor, daß zur Steuerung des pH-Wertes der verdünnten Wasser-/Elektrolytlösung in dem Anodenraum des Elektrolysereaktors auf einen pH-Wert zwischen 2,7 und 3,5, insbesondere zwischen 2,7 und 3,3, bei einem vorgegebenen Sollstrom zwischen den Elektroden des Elektrolysereaktors eine von der jeweiligen Zusammensetzung des zu desinfizierenden Wassers abhängige Verweilzeit (Tᵥ) und/oder eine von der jeweiligen Zusammensetzung des Wassers abhängiger Volumenstrom (V') der verdünnten Wasser-/Elektrolytlösung in dem bzw. durch den Elektrolysereaktor ermittelt wird. Dabei sieht eine vorteilhafte Ausführungsform des Verfahrens vor, daß die Verweilzeit (Tᵥ) und/oder der Volumenstrom (V') in dem bzw. durch den Elektrolysereaktor in Abhängigkeit von der Leitfähigkeit (κ) und/oder von der Härte (H) des Wassers ermittelt wird. Da insbesondere die Leitfähigkeit des Wassers einen verhältnismäßig großen Einfluß auf die zum Erreichen des erfindungsgemäßen pH-Wertes erforderliche Verweilzeit der verdünnten Wasser-/Elektrolytlösung in dem Reaktor bzw. - im Falle einer (semi)kontinuierlichen Verfahrensführung - auf den entsprechenden Volumenstrom durch den Reaktor besitzt, ist es auf diese Weise möglich, zunächst einen für das jeweils zu desinfizierende Wasser zweckmäßige(n) Verweilzeit/Volumenstrom zu ermitteln, wobei zur Ermittlung dieser Abhängigkeit ein geeigneter Strom an die Elektroden des Reaktors angelegt wird, welcher z.B. empirisch ermittelt werden kann und vorzugsweise - wie auch die Menge an zudosierter Elektrolytlösung - konstant gehalten wird. Dabei kann vorgesehen sein, daß die Verweilzeit bzw, der Volumenstrom anstelle in Abhängigkeit von der Leitfähigkeit des zu desinfizierenden Wassers in Abhängigkeit von der für die elektrische Leitfähigkeit des Wassers repräsentativen Härte des Wassers ermittelt wird, d.h. anstelle der zur Gesamtkonzentration an in dem Wasser enthaltenen Ionen proportionalen Leitfähigkeit des Wassers wird nur dessen Härte, also dessen Konzentration an Calcium- und Magnesiumionen, herangezogen.

Eine bevorzugte Ausführung sieht in diesem Zusammenhang vor, daß die Verweilzeit (Tᵥ) und/oder der Volumenstrom (V') gemäß einer Geradengleichung der Form

Tᵥ = k₁ · K + k₂

und/oder

V' = k₃ · H + k₄

ermittelt wird, wobei k₁, k₂, k₃ und k₄ reaktorspezifische Konstanten sind. Die genannten Geradengleichungen können z.B. auf einfache Weise dadurch ermittelt werden, indem Wässer mit verschiedener Leitfähigkeit bei einer bestimmten Verweilzeit in dem bzw. mit einem bestimmten Volumenstrom durch den Reaktor und unter einer bestimmten - insbesondere konstanten - Zudosierung an Elektrolytlösung und bei einem bestimmten - insbesondere konstanten - Stromfluß zwischen den Elektroden des Elektrolysereaktors elektrochemisch aktiviert werden und dabei der pH-Wert auf den erfindungsgemäßen Wertebereich gesteuert wird, wonach die hierbei für die verschiedenen Wässer erforderlichen Verweilzeiten bzw. Volumenströme in Abhängigkeit der elektrischen Leitfähigkeit des Wassers aufgetragen werden und der Reaktor mit einem dementsprechenden Volumenstrom (bzw. Verweilzeit) betrieben wird.

Wie bereits erwähnt, wird die Menge an zudosierter Elektrolytlösung sowohl hinsichtlich der Voreinstellung des Reaktors als auch während des Betriebs vorzugsweise im wesentlichen konstant gehalten, wobei der Reaktor z.B. stets in seinem optimalen Betriebszustand mit einem bestimmten Volumenstrom an Wasser (bzw. mit einer bestimmten Verweilzeit des Wassers in dem Reaktor) gefahren werden kann und z.B. im Falle einer Desinfektion von Wasser mit Spitzenlasten großen Bedarfs und Ruhezeiten geringen Bedarfs vorgesehen sein kann, den Reaktor einerseits von Zeit zu Zeit abzuschalten (z.B. während Ruhezeiten) und andererseits einen Speicher für die produzierte elektrochemisch aktivierte, anodische, verdünnte Wasser-/Elektrolytlösung bereitzustellen, um Spitzenlasten zu überbrücken.

Eine vorteilhafte Ausführungsform des erfindungsgemäßen Verfahrens sieht vor, daß die Elektrolytlösung in Form einer im wesentlichen reinen Alkalimetallchloridlösung, insbesondere in Form einer Natrium- (NaCl) und/oder einer Kaliumchloridlösung (KCl), zugesetzt wird. Dabei kann die Elektrolytlösung in bevorzugter Ausführung in Form einer im wesentlichen gesättigten Alkalimetallchloridlösung zugesetzt werden. Um für eine hohe Reproduzierbarkeit zu sorgen, sollte die Alkalimetallchloridlösung in hoher Reinheit eingesetzt werden, d.h. sie sollte insbesondere im wesentlichen frei sein von anderen Halogenidionen, also solchen aus der Gruppe Bromid (Br⁻) , Fluorid (F⁻) und Iodid (I⁻), von Oxohalogenidionen, wie Hypochlorit (ClO⁻), Chlorit (ClO₂⁻), Chlorat (ClO₃⁻), Perchlorat (ClO₄⁻), Bromat (BrO₃⁻) etc. Ferner sollte sie im wesentlichen frei von Schwermetallen sein, wie insbesondere solchen aus der Gruppe Antimon (Sb), Arsen (As), Blei (Pb), Cadmium (Cd), Chrom (Cr), Nickel (Ni), Quecksilber (Hg), Selen (Se), Eisen (Fe) und Mangan (Mn), sowie vorzugsweise im wesentlichen keine härtebildenden Erdalkalimetalle, wie insbesondere Calcium (Ca) und Magnesium (Mg), enthalten.

Die spezifische elektrische Leitfähigkeit der Elektrolytlösung (vor dem Zudosieren derselben zu dem elektrochemisch zu aktivierenden Wasser) kann bevorzugt auf einen Wert zwischen etwa 1, 5 _{·} 10⁵ µS/cm und etwa 3, 5 · 10⁵ µS/cm, insbesondere zwischen etwa 1,8 · 10⁵ µS/cm und etwa 2,8 · 10⁵ µS/cm, vorzugsweise zwischen etwa 2,0 · 10⁵ µS/cm und etwa 2,5 · 10⁵ µS/cm, eingestellt werden.

Die Elektrolytkonzentration, insbesondere die Alkalimetallchloridkonzentration, der dem Elektrolysereaktor zugesetzten, verdünnten Wasser-/Elektrolytlösung (also nach dem Zudosieren der Elektrolytlösung in das elektrochemisch zu aktivierende Wasser), insbesondere der Wasser-/Alkalimetallchloridlösung, sollte, wie eingangs erwähnt, einen Wert von etwa 20 g/l grundsätzlich nicht wesentlich überschreiten. In vorteilhafter Ausgestaltung sollte deren Wert zwischen etwa 0,1 g/l und etwa 10 g/l, insbesondere zwischen etwa 0,1 g/l und etwa 5 g/l, vorzugsweise zwischen etwa 0,1 g/l und etwa 3 g/l, (jeweils Gramm pro Liter Elektrolyt bzw. Alkalimetallchlorid) gesteuert werden. Eine solche Konzentration hat sich für eine optimale Desinfektions- und Depotwirkung der elektrochemisch aktivierten, verdünnten Wasser-/Elektrolyt- bzw. Wasser-/Alkalimetallchloridlösung als geeignet erwiesen und ermöglicht ferner eine Einstellung eines als günstig gefundenen pH-Wertes der elektrochemisch aktivierten, anodischen, verdünnten Wasser-/Elektrolytlösung am Austritt aus dem Elektrolysereaktor im Bereich um 3 sowie ein Redoxpotential im Bereich um 1340 mV vs. SHE (Standardwasserstoffelektrode).

Eine Weiterbildung des erfindungsgemäßen Verfahrens, welche im übrigen auch als solche, d.h. ohne die Steuerung des pH-Wertes der verdünnten Wasser-/Elektrolytlösung in dem Anodenraum des Elektrolysereaktors, bereits zu einer merklichen Verbesserung der elektrochemischen Aktivierung von Wasser führt, sieht vor, daß die spezifische elektrische Leitfähigkeit des elektrochemisch zu aktivierenden Wassers vor Zusetzen der Elektrolytlösung auf einen Wert von höchstens 350 µS/cm eingestellt wird. Überraschenderweise wurde gefunden, daß durch eine solche Einstellung einer spezifischen elektrischen Leitfähigkeit des eingesetzten Wassers bzw. des Rohwassers auf einen Wert von höchstens etwa 350 µS/cm, vorzugsweise auf einen Wert zwischen etwa 0,055 µS/cm und etwa 150 µS/cm und insbesondere auf einen Wert zwischen etwa 0,055 µS/cm und etwa 100 µS/cm, vor dem Zusetzen der Elektrolytlösung (was die Leitfähigkeit des eingesetzten Wassers in aller Regel ohnehin um ein Vielfaches erhöht), eine noch bessere Reproduzierbarkeit des Verfahrens hinsichtlich der Desinfektions- und Depotwirkung des Desinfektionsmittels in Form der anodischen, elektrochemisch aktivierten, verdünnten Wasser-/Elektrolytlösung sichergestellt wird, und zwar praktisch unabhängig von dem eingesetzten Wasser. Eine solche "Standardisierung" des eingesetzten Rohwassers ermöglicht nicht nur eine besonders einfache Einstellung der Verfahrensparameter, wie Elektrodenspannung bzw. -strom, Verweilzeit der verdünnten Wasser-/Elektrolytlösung in dem Elektrolysereaktor, Menge an zudosierter Elektrolytlösung etc., sondern macht auch auf einfache Weise einen Einsatz von Wässern mit praktisch beliebiger Zusammensetzung ohne jegliche Beeinträchtigung des erhaltenen Desinfektionsmittels möglich, wodurch eine höchst zuverlässige potentialkontrollierte anodische Oxidation der verdünnten Wasser-/ Elektrolytlösung im Anodenraum des Elektrolysereaktors gewährleistet wird.

Darüber hinaus lassen sich in dem elektrochemisch zu aktivierenden Wasser gegebenenfalls enthaltene Ionen, welche bei der elektrochemischen Aktivierung - wenn auch in nur geringen Konzentrationen - in gesundheitlich bedenkliche Stoffe umgewandelt werden, zumindest weitestgehend elimieren. Als Beispiel seien Bromidionen erwähnt, welche - wie auch bei der bei der Trinkwasseraufbereitung häufig durchgeführten Ozonierung - zu Bromat oxidiert werden können, welches in höheren Konzentrationen eine cancerogene Wirkung besitzt. In der Praxis kann das dem Elektrolysereaktor zugeführte Prozeßwasser beispielsweise stromauf der Zudosierung der Elektrolytlösung mittels einer vorzugsweise kontinuierlich arbeitenden Leitfähigkeitsmeßzelle oder -elektrode auf seine spezifische elektrische Leitfähigkeit untersucht werden und - je nach Wasserbeschaffenheit - bedarfsweise oder stetig deionisiert bzw. demineralisiert werden, wie es weiter unten noch näher erläutert ist.

Mit "spezifischer elektrischer Leitfähigkeit" des Wassers bzw. der verdünnten Wasser-/Elektrolytlösung ist im Sinne der Erfindung im übrigen die spezifische ionische Leitfähigkeit angesprochen, welche auf der Leitfähigkeit des Wassers bzw. der Wasser-/Elektrolytlösung aufgrund der hierin gelösten, bewegungsfähigen Ionen beruht.

Gemäß einer bevorzugten Ausführungsform kann vorgesehen sein, daß die Härte des elektrochemisch zu aktivierenden Wassers vor Zusetzen der Elektrolytlösung auf einen Wert zwischen etwa 0 und etwa 12°dH, insbesondere zwischen etwa 0 und etwa 4 dH, vorzugsweise zwischen etwa 0 und etwa 2°dH, z.B. zwischen etwa 1°dH und 2°dH, eingestellt wird. Mit "Härte" ist in diesem Zusammenhang die Konzentration von zweiwertigen Erdalkalimetallionen, also Calcium (Ca), Magnesium (Mg), Strontium (Sr) und Barium (Ba), wobei die beiden letztgenannten in der Praxis in der Regel keine Rolle spielen. 1°dH entspricht einer Konzentration von Erdalkalimetallionen von 0,179 mMol/l, 2°dH einer Konzentration von 0,358 mMol/l etc. Eine solche Vorgehensweise ist insbesondere bei relativ harten, calcium- und/oder magnesiumhaltigen Wässern zweckmäßig, um die Lebensdauer des Elektrolysereaktors zu erhöhen bzw. dessen Wartungsintervalle zu verlängern. Indes sollte insbesondere bei sehr leitfähigen Wässern, d.h. bei solchen mit einer hohen Gesamtionenkonzentration, dafür Sorge getragen werden, das Wasser nicht lediglich mittels eines Ionentauschers zu enthärten, da der Ionentauscher je ein zweiwertiges Erdalkalimetallion durch zwei einwertige Alkalimetallionen ersetzt und somit die Leitfähigkeit insgesamt weiter erhöht wird. Aus diesem Grund kann es zweckmäßig sein, das Wasser zunächst zu enthärten und sodann die Leitfähigkeit auf einen Wert innerhalb des erfindungsgemäßen Bereiches abzusenken.

Darüber hinaus kann es insbesondere bei organisch belasteten oder eutrophierten Wässern von Vorteil sein, daß der gesamte organische Kohlenstoffanteil (TOC, total organic carbon) des elektrochemisch zu aktivierenden Wassers auf einen TOC-Wert von höchstens etwa 25 ppb (parts per billion; Teile pro Milliarde), insbesondere von höchstens etwa 20 ppb, vorzugsweise von höchstens etwa 15 ppb, eingestellt wird. Entsprechendes kann hinsichtlich des chemischen Sauerstoffbedarfs (COD, chemical oxygen demand) gelten, welcher in vorteilhafter Ausgestaltung auf einen COD-Wert von höchstens etwa 7 mg O₂/l, insbesondere von höchstens etwa 5 mg O₂/l, vorzugsweise von höchstens etwa 4 mg O₂/l, eingestellt werden kann.

Zur Einstellung bzw. Erniedrigung der spezifischen elektrischen Leitfähigkeit und/oder der Härte des elektrochemisch zu aktivierenden Wassers haben sich z.B. Membranverfahren, wie Umkehrosmose, Mikro-, Nano-, Ultrafiltration oder dergleichen, als geeignet erwiesen, wobei selbstverständlich auch andere zu diesem Zweck bekannte Verfahren zum Einsatz kommen können. Zur Einstellung bzw. Verringerung des gesamten organischen Kohlenstoffanteils (TOC) und/oder des chemischen Sauerstoffbedarfs (COD) des elektrochemisch zu aktivierenden Wassers kommen z.B. Oxidationsverfahren, insbesondere mittels elektromagnetischer Strahlung im ultravioletten Bereich (UV-Strahlung), oder auch andere bekannte Verfahren in Betracht.

Die Steuerung der Elektrolytkonzentration, insbesondere der Alkalimetallchloridkonzentration, der dem Elektrolysereaktor zugesetzten, verdünnten Wasser-/Elektrolytlösung, insbesondere der Wasser-/Alkalimetallchloridlösung, geschieht vorzugsweise durch Steuerung der dem elektrochemisch zu aktivierenden Wasser zugesetzten Menge an Elektrolytlösung, beispielsweise mittels einer Dosierpumpe. Um für eine homogene Konzentrationsverteilung zu sorgen, wird das elektrochemisch zu aktivierende Wasser zweckmäßig nach Zudosieren der Elektrolytlösung innig durchmischt.

Gemäß einer bevorzugten Ausführungsform kann vorgesehen sein, daß die Steuerung der Alkalimetallchloridkonzentration der dem Elektrolysereaktor zugesetzten, verdünnten Wasser-/Alkalimetallchloridlösung in Abhängigkeit der hiermit korrespondierenden spezifischen elektrischen Leitfähigkeit der dem Elektrolysereaktor zugesetzten, verdünnten Wasser-/Alkalimetallchloridlösung durchgeführt wird, wobei die Abhängigkeit der Alkalimetallchloridkonzentration von der spezifischen elektrischen Leitfähigkeit der dem Elektrolysereaktor zugesetzten, verdünnten Wasser-/Alkalimetallchloridlösung für das jeweils eingesetzte, elektrochemisch zu aktivierende Wasser vorab ermittelt wird. Nachdem diese Abhängigkeit in Form einer Kalibrierkurve ermittelt worden ist, muß folglich nur die für die Alkalimetallchloridkonzentration der verdünnten Wasser-/Alkalimetallchloridlösung repräsentative Leitfähigkeit gemessen und anhand der Kalibrierkurve in die Alkalimetallchloridkonzentration umgerechnet werden. In Kenntnis der Konzentration der zur Verfügung stehenden Alkalimetallchloridlösung kann somit die jeweils erforderliche Menge zudosiert werden, um die gewünschte Konzentration zu erhalten, was zweckmäßig mittels einer elektronischen Datenverarbeitungseinheit erfolgt, welche einerseits mit einer Leitfähigkeitsmeßzelle oder -elektrode und andererseits mit einem entsprechenden Dosierorgan in Verbindung steht.

Dabei hat es sich insbesondere als zweckmäßig erwiesen, wenn die Abhängigkeit der Alkalimetallchloridkonzentration von der spezifischen elektrischen Leitfähigkeit der dem Elektrolysereaktor zugesetzten, verdünnten Wasser-/Alkalimetallchloridlösung gemäß einer Kalibriergeraden der Form

κ_{ges} = κ_{w} + dκ/d[MeCl] · [MeCl]

ermittelt wird, wobei κ_{ges} die spezifische elektrische Leitfähigkeit der dem Elektrolysereaktor zugesetzten, verdünnten Wasser-/Alkalimetallchloridlösung, κ_{w} die spezifische Leitfähigkeit des jeweils eingesetzten, zu desinfizierenden Wassers (unmittelbar vor dem Zusetzen der Elektrolytlösung, vorzugsweise höchstens 350 µS/cm), [MeCl] die Alkalimetallchloridkonzentration der dem Elektrolysereaktor aufgegebenen, verdünnten Wasser-/Alkalimetallchloridlösung und dκ/d[NaMe] die wasserspezifische Steigung der Kalibriergeraden ist, d.h. die Konstante dκ/d[MeCl] hängt von den Inhaltsstoffen des eingesetzten Wassers ab, dessen spezifische elektrische Leitfähigkeit zum Zeitpunkt der Zudosierung der Elektrolytlösung, wie bereits erwähnt, bereits auf einen Wert von höchstens etwa 350 µS/cm eingestellt worden sein kann. Zur Durchführung einer solchen Kalibrierung kann beispielsweise für eine bestimmte bekannte Konzentration der auf Vorrat gehaltenen, dem Wasser zuzudosierenden Alkalimetallchloridlösung bei einer bekannten - z.B. gemessenen - Leitfähigkeit κ_{w} des elektrochemisch zu aktivierenden Wassers die Gesamtleitfähigkeit κ_{ges} der verdünnten Wasser-/Alkalimetallchloridlösung bei verschiedenen Mengen an zugesetzter Alkalimetallchloridlösung gemessen werden. Trägt man diese gemessenen Werte der Gesamtleitfähigkeit κ_{ges} der verdünnten Wasser-/Alkalimetallchloridlösung auf der Ordinate gegenüber der Alkalimetallchloridkonzentration [MeCl] der verdünnten Wasser-/Alkalimetallchloridlösung auf der Abszisse auf, so erhält man die Kalibriergerade, bei welcher der Faktor dκ/d[MeCl] die Geradensteigung und der Wert κ_{W} den Ordinatenschnittpunkt dieser Gerade repräsentiert. Wie bereits erwähnt, kann [MeCl] dabei vorzugsweise z.B. [NaCl] und/oder [KCl] sein.

Wie bereits angedeutet, kann es nicht nur im Hinblick auf die Herstellung des erfindungsgemäßen Desinfektionsmittels selbst, sondern auch im Falle einer Desinfektion von Wasser mittels eines solchen Desinfektionsmittels von Vorteil sein, ausschließlich die in dem Anodenraum erzeugte, elektrochemisch aktivierte, verdünnte Wasser-/Elektrolytlösung zu verwenden und die zur Desinfektion weniger geeignete, in dem Kathodenraum erzeugte verdünnte Wasser-/Elektrolytlösung zu verwerfen.

Ferner kann es zu Desinfektionszwecken von Wasser oder auch von beliebigen anderen Medien zweckmäßig sein, wenn das Desinfektionsmittel in im wesentlichen reiner Form oder in Form einer Verdünnung von bis zu 1:500, insbesondere von bis zu 1:400, Anteilen eines Verdünnungsmittels, insbesondere Wasser, eingesetzt wird.

Neben der reinen Herstellung des Desinfektionsmittels selbst kann das erfindungsgemäße Verfahren insbesondere zur Desinfektion von Wasser, wie Trink- und Brauchwasser, Regenwasser, Schwimmbadwasser, Industriewasser und -abwasser und dergleichen, eingesetzt werden. In diesem Zusammenhang ist es beispielsweise in verfahrenstechnischer Hinsicht günstig, wenn aus dem zu desinfizierenden Wasser ein Teilstrom abgezweigt, der Teilstrom elektrochemisch aktiviert und zumindest (oder ausschließlich) der in dem Anodenraum elektrochemisch aktivierte Teilstrom dem zu desinfizierenden Wasser als Desinfektionsmittel zugesetzt wird, wobei dieses Desinfektionsmittel - wie bereits erwähnt - je nach Anwendungsfall dem zu desinfizierenden Wasser in geeigneter Verdünnung wieder zugesetzt wird.

Schließlich ist das erfindungsgemäße Verfahren insbesondere zur kontinuierlichen oder semikontinuierlichen Durchführung geeignet, wobei ein Teilstrom des zu desinfizierenden Wassers bzw. das zur Herstellung des Desinfektionsmittels in Form der in erfindungsgemäßer Weise elektrochemisch aktivierten, anodischen, verdünnten Wasser-/Elektrolytlösung (semi)kontinuierlich durch den Elektrolysereaktor hindurch geleitet wird.

Durch das erfindungsgemäße Verfahren läßt sich folglich ein Desinfektionsmittel in Form einer elektrochemisch aktivierten, anodischen, verdünnten Wasser-/Elektrolytlösung (Anolyt) herstellen, dessen pH-Wert zwischen etwa 2,7 und etwa 3;5, vorzugsweise zwischen etwa 2,7 und etwa 3,3, insbesondere zwischen etwa 2,8 und etwa 3,2, und dessen Redoxpotential in vorteilhafter Ausführung zwischen etwa 1240 mV und etwa 1360 mV, vorzugsweise zwischen etwa 1280 mV und etwa 1360 mV, insbesondere zwischen etwa 1320 mV und etwa 1360 mV (jeweils bezogen auf die SHE), beträgt.

Die gemäß dem erfindungsgemäßen Verfahren hergestellte, elektrochemisch aktivierte, anodische, verdünnte Wasser-/Elektrolytlösung kann als Desinfektionsmittel z.B. überall dort zum Einsatz gelangen, wo eine einwandfreie Desinfektion von Wasser, insbesondere auch unter Einhaltung der Trinkwasserverordnung, erforderlich ist, wie zur Desinfektion der kommunalen Wasserversorgung oder der Wasserversorgung von Krankenhäusern, Schulen, Pflegeheimen, Gewerbebetrieben, Hotels oder anderen gastronomischen Betrieben und Sportvereinen (z.B. zur Zudosierung in das Wasser der sanitären Anlagen), Bahnhöfen, Flughäfen, Großküchen, zur Desinfektion von Schwimmbadwasser oder Regenwasser (z.B. zur Beigabe bei der Regenwasseraufbereitung) bzw. zum Zusatz in Wasservorratstanks beliebiger Art, für Entsalzungsanlagen, wie Meerwasserentsalzungsanlagen auf Schiffen oder auf dem Binnenland, zur Verhinderung der Verschleppung von Keimen im Wasser von Textilwaschmaschinen, zur schnellen Entfärbung von Färbereiabwässern, für praktisch beliebige Industrie(ab)wässer, wie zur Beimischung in Kühlwasser (beispielsweise für Dreh-, Fräs-, Bohr-, Schneide- oder andere Werkzeugmaschinen), für Klimaanlagen und Luftbefeuchtungssysteme, für Osmoseanlagen, als Zusatz zu dem Wasser zum Anmachen von Beton und Zement, als Zusatz zu dem Wasser bei der Herstellung elektronischer Bauelemente und Schaltungen, als Zugabe in das Wasser von Schnittblumen, zur Zudosierung in das Tränke- und Abwasser von Tierhaltungsbetrieben und Schlachthöfen bzw. zur Desinfektion von in diesem Zusammenhang eingesetzten Vorrichtungen, wie Brutmaschinen, Melkmaschinen und dergleichen, etc. In allen Fällen wird zur akuten Desinfektion oder während des normalen Betriebs eine zuverlässige Desinfektion erreicht und wird die unerwünschte Bildung von Algen und/oder Schlämmen verhindert. Das Desinfektionsmittel kann entweder in im wesentlichen reiner Form oder - insbesondere bei der Wasseraufbereitung - in Form einer Verdünnung von bis zu etwa 1:500, vorzugsweise bis zu etwa 1:400, Anteilen eines Verdünnungsmittels, wie Wasser, eingesetzt werden, wobei sich im Falle der Wasseraufbereitung z.B. eine Verdünnung im Bereich von etwa 1:400 in vielen Fällen als zweckmäßig erwiesen hat.

Das Desinfektionsmittel in Form der erfindungsgemäß elektrochemisch aktivierten, anodischen Wasser-/Elektrolytlösung kann darüber hinaus - ebenfalls z.B. in reiner Form oder insbesondere in Form einer jeweils geeigneten Verdünnung - zur Desinfektion von Lebensmitteln, wie Getreide oder Mehl, Gewürze, Obst, Gemüse, Speiseeis und als Kühlmittel dienendes Eis, z.B. bei der Lagerung von Fleisch, Fischen und Meeresfrüchten anläßlich Transport und Verkauf, Tierprodukten und dergleichen, verwendet werden, wobei eine einwandfreie Abtötung von Keimen, wie Fäulnisbakterien etc., und somit bei einer sehr guten Gesundheitsverträglichkeit eine längere Lagerbeständigkeit erzielt wird.

Des weiteren ist eine Verwendung eines erfindungsgemäß hergestellten Desinfektionsmittels zur Desinfektion von Saatgut geeignet, wobei es z.B. als Silage- und Konservierungsmittel bei der Einlagerung von Saatgut und Getreide in Silos eingesetzt werden kann.

Ein weiteres bevorzugtes Einsatzgebiet eines solchen Desinfektionsmittels besteht in der Desinfektion von Verpakkungsbehältern und Verpackungen, insbesondere für hygienische Produkte, wie Lebensmittel, Pharmazeutika, sterile Gegenstände (wie Spritzen, Operationsbesteck etc.) und dergleichen.

Überdies kann eine Verwendung eines solchen Desinfektionsmittels für Reaktionsmedien zur Durchführung von Lösungsmittel- und Emulsionspolymerisationen günstig sein, wobei der Einsatz von hierfür erforderlichen Emulgatoren verringert wird und die Polymerisationsrate überraschenderweise gesteigert werden kann, wie es sich bei einem Experiment anläßlich der Herstellung von Divinylstyrolkautschuk gezeigt hat.

Ferner besteht ein weiteres bevorzugtes Anwendungsgebiet eines solchen Desinfektionsmittels als Additiv für insbesondere wasserlösliche Farben, Lacke und Pigmente, welchen eine biocide Wirkung verliehen werden kann, sowie als Additiv für Kühl- und Schmiermittel, z.B. für industrielle Kühlkreisläufe oder für technische Schmiermittel auf der Basis von Wasser, Öl oder Fett.

Schließlich kann ein solches Desinfektionsmittels auch als Additiv für Treib- und Kraftstoffe, wie Heizöl, Benzin, Kerosin und dergleichen, verwendet werden.

In allen Fällen kann das Desinfektionsmittel in Form der in erfindungsgemäßer Weise elektrochemisch aktivierten, anodischen Wasser-/Elektrolytlösung bei geeigneter Lagerung (insbesondere im wesentlichen unter Sauerstoffabschluß) problemlos bis zu etwa sechs Monaten auf Vorrat gehalten werden.

Nachstehend ist das erfindungsgemäße Verfahren anhand von Ausführungsbeispielen eines Verfahrens zur Aufbereitung bzw. Desinfektion von Trinkwasser unter Bezugnahme auf die Zeichnungen näher erläutert. Dabei sei darauf hingewiesen, daß die Herstellung der elektrochemisch aktivierten, anodischen, verdünnten Wasser-/Elektrolytlösung in reiner oder andersartig verdünnter Form in einem identischen Elektrolysereaktor geschehen kann. In den Zeichnungen zeigen:
- Fig. 1: ein schematisches Fließbild einer ersten Ausfüh- rungsform eines erfindungsgemäßen Verfahrens zur Desinfektion von Wasser durch elektrochemische Aktivierung (ECA);
- Fig. 2: eine geschnitten dargestellte Detailansicht des Elektrolysereaktors gemäß Fig. 1;
- Fig. 3: eine geschnitten dargestellte Detailansicht des Mischers gemäß Fig. 1, und
- Fig. 4: ein schematisches Fließbild einer zweiten Ausfüh- rungsform eines erfindungsgemäßen Verfahrens zur Desinfektion von Wasser durch elektrochemische Aktivierung (ECA), welche sich von der Ausfüh- rungsform gemäß Fig. 1 insbesondere durch den Einsatz einer dem Elektrolysereaktor vorgeschal- teten Reinwasseranlage unterscheidet.

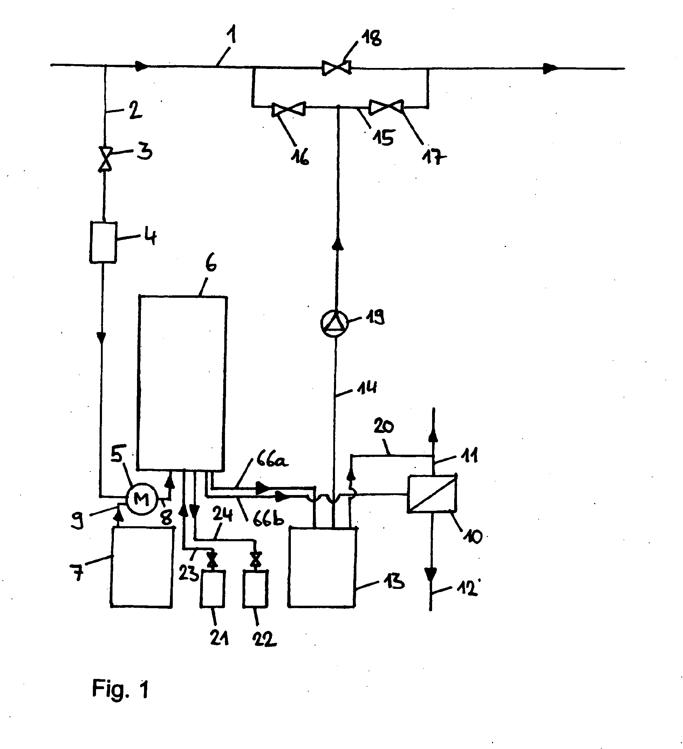

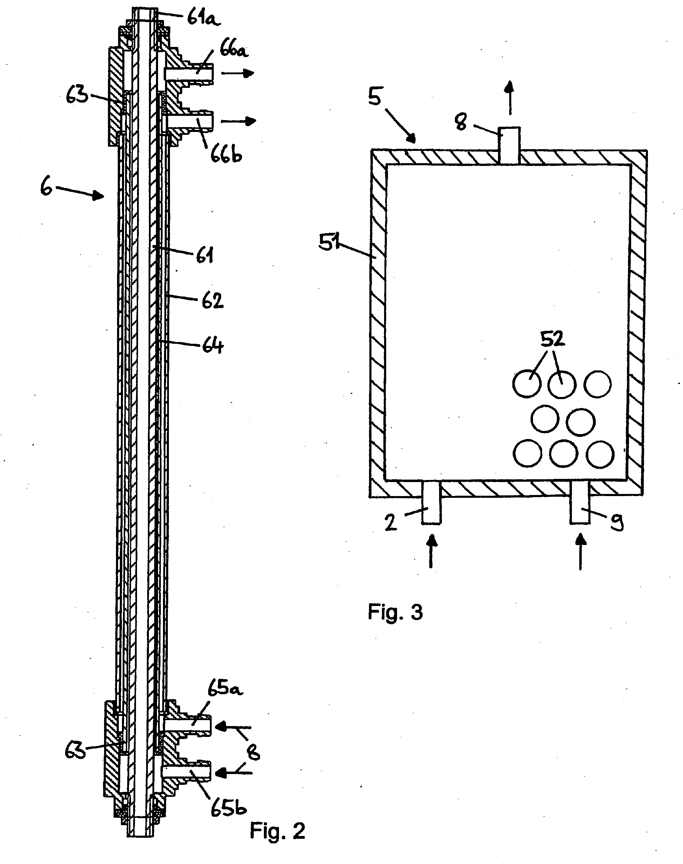

Die in Fig. 1 schematisch dargestellte, zur kontinuierlichen oder semikontinuierlichen Durchführung eines erfindungsgemäßen Verfahrens geeignete Vorrichtung zur Desinfektion von Wasser durch elektrochemische Aktivierung (ECA) unter potentialkontrollierter anodischer Oxidation (PAO) umfaßt eine Hauptwasserleitung 1, in welcher das zu desinfizierende Wasser gefördert wird. Die Hauptwasserleitung 1 kann beispielsweise von einer Zulaufleitung der Wasserversorgung eines Krankenhauses, eines Gewerbebetriebs, eines Hotels oder eines anderen gastronomischen Betriebs, von der Umwälzleitung eines Swimmingpools oder dergleichen gebildet sein. An die Hauptwasserleitung 1 ist eine Abzweigleitung 2 angeschlossen, welche mit einem Ventil 3, insbesondere in Form eines Steuerventils, sowie mit einem Filter 4, insbesondere in Form eines Feinfilters mit einer Lochweite von beispielsweise etwa 80 bis 100 µm, ausgestattet ist und über einen weiter unten unter Bezugnahme auf Fig. 3 näher erläuterten Mischer 5 in einen ebenfalls weiter unten unter Bezugnahme auf Fig. 2 näher beschriebenen Elektrolysereaktor 6 mündet. Über die Abzweigleitung 2 ist somit ein mittels des Steuerventils 3 steuerbarer Teilstrom des in der Hauptwasserleitung 1 geförderten Wassers in den Elektrolysereaktor 6 überführbar, wobei z.B. ein Teilstrom des in der Hauptwasserleitung 1 geförderten Wassers in der Größenordnung von 1/200 über die Abzweigleitung 2 abgezweigt wird.

Der Mischer 5 steht zulaufseitig einerseits mit der Abzweigleitung 2, andererseits mit einem Reservoir 7 zur Aufnahme einer Elektrolytlösung - hier z.B. einer im wesentlichen gesättigten Natriumchloridlösung - in Verbindung, welche in dem Mischer 5 möglichst homogen miteinander vermischt werden und über eine gemeinsame, ablaufseitige Leitung 8 des Mischers 5 in den Elektrolysereaktor 6 gelangen. Die von dem Reservoir 7 in den Mischer 5 führende Leitung 9 ist ferner mit einer in Fig. 1 nicht dargestellten Dosierpumpe ausgestattet, um dem in der Abzweigleitung 2 geförderten Wasser eine definierte Menge an Elektrolytlösung zuzusetzen. Wie insbesondere aus Fig. 3 ersichtlich, ist der Mischer 5 beim vorliegenden Ausführungsbeispiel von einem Kugelmischer gebildet, welcher eine konstant gleichmäßige Durchmischung des Wassers mit der Elektrolytlösung sicherstellt. Er umfaßt im wesentlichen einen etwa zylindrischen Behälter 51, an dessen entgegengesetzten Enden die Zuläufe 2, 9 bzw. der Ablauf 8 angeschlossen sind und in welchem eine Schüttung aus in Fig. 3 exemplarisch angedeuteten Kugeln 52 oder anderem Schüttgut angeordnet ist, durch welches das Wasser und die Elektrolytlösung hindurch fließen, wobei die Kugeln 52 in Schwingungen angeregt werden und dabei eine sehr homogene Durchmischung des Wassers mit der diesem zugesetzten Elektrolytlösung gewährleisten.

Wie insbesondere der Fig. 2 zu entnehmen ist, umfaßt der Elektrolysereaktor 6 eine Anode 61, welche beim vorliegenden Ausführungsbeispiel z.B. von einem mit katalytisch wirksamem Rutheniumdioxid (RuO₂) beschichteten Hohlrohr aus Titan gebildet ist und an welches endseitig über ein Außengewinde 61a der Pluspol einer nicht näher dargestellten Spannungsquelle anschließbar ist. Alternativ oder zusätzlich zu Rutheniumoxid kann beispielsweise auch eine Beschichtung auf der Basis von Iridiumdioxid (IrO₂) oder einer Mischung beider (RuO₂/IrO₂) oder anderer Oxide, wie Titandioxid (TiO₂), Bleidioxid (PbO₂) und/oder Mangandioxid (MnO₂), vorgesehen sein. Der Elektrolysereaktor 6 umfaßt des weiteren eine Kathode 62, welche zweckmäßig aus Edelstahl oder ähnlichen Materialien, wie Nickel (Ni), Platin (Pt), etc., gefertigt und beim vorliegenden Ausführungsbeispiel ebenfalls von einem Hohlrohr gebildet ist, innerhalb dessen die Anode 61 koaxial angeordnet ist. Die Kathode 62 ist mittels z.B. sie außenseitig umgreifender Klemmen (nicht dargestellt) an den Minuspol der nicht näher weitergegebenen Spannungsquelle anschließbar. Koaxial zu der Anode 61 sowie zu der Kathode 62 und zwischen diesen ist ein mittels Dichtringen 63 abgedichtetes, rohrförmiges Diaphragma 64 angeordnet, welches den zwischen der Anode 61 und der Kathode 62 befindlichen, ringförmigen Reaktionsraum in einen Anodenraum und in einen Kathodenraum abtrennt. Das Diaphragma 64 verhindert eine Vermischung der im Anodenraum und Kathodenraum befindlichen Flüssigkeit und läßt jedoch einen Stromfluß zu, welcher insbesondere für die Migration von Ionen keinen großen Widerstand darstellt. Das Diaphragma 64 ist beim vorliegenden Ausführungsbeispiel z.B. aus elektrisch - bzw. ionisch - leitfähigem, aber im wesentlichen flüssigkeitsdichtem, porösem Zirkoniumdioxid (ZrO₂) gebildet. Andere Materialien mit einem verhältnismäßig geringen Widerstand, wie Aluminiumoxid (Al₂O₃), Ionenaustauschmembranen, insbesondere solche auf Kunststoffbasis etc., können gleichfalls eingesetzt werden.

Der Elektrolysereaktor 6 besitzt ferner zwei Einlässe 65a, 65b, über welche die aus dem Mischer 5 über die Leitung 8 austretende Wasser-/Elektrolytlösung in den Reaktionsraum des Reaktors 6, d.h. in dessen Anodenraum und in dessen von diesem durch das Diaphragma 64 räumlich getrennten Kathodenraum, eingespeist wird. Ein hierfür vorgesehener, z.B. etwa T-förmiger Abzweig ist in Fig. 1 nicht gezeigt. Wie wiederum insbesondere aus Fig. 3 und überdies aus Fig. 1 ersichtlich, weist der Elektrolysereaktor 6 ferner zwei Auslässe 66a, 66b auf, über welche die Wasser-/Elektrolytlösung nach der chemischen Aktivierung in dem Reaktor 6 aus diesem abführbar ist. Während der Auslaß 66a zum Abführen der elektrochemisch aktivierten Wasser-/Elektrolytlösung aus dem Anodenraum des Reaktors 6 - d.h. zum Abführen des sogenannten "Anolyts" - dient, dient der Auslaß 66b zum Abführen aus dem Kathodenraum - d.h. zum Abführen des sogenannten "Katholyts". Ferner kann vorgesehen sein, daß beim Anfahren des Elektrolysereaktors 6 über einen bestimmten Zeitraum auch der "Anolyt", d.h. die elektrochemisch aktivierte, anodische Wasser-/Elektrolytlösung, verworfen wird, um anfängliche Qualitätsbeeinträchtigungen, so lange der Elektrolysereaktor 6 noch nicht seinen gewünschten Betriebszustand erreicht hat, auszuschließen.

Nachfolgend sind die geometrischen Abmessungen des vorliegend eingesetzten Elektrolysereaktors 6 in Form einer Auflistung wiedergegeben:
Länge des Kathodenraumes: 18,5 cm;
Volumen des Kathodenraumes: 10 ml;
Fläche der Kathode: 92,4 cm²;
Länge des Anodenraumes: 21,0 cm;
Volumen des Anodenraumes: 7 ml;
Fläche der Anode: 52,7 cm²;
Abstand zwischen Kathode und Anode: ca. 3 mm (einschließlich Diaphragma).

Der Elektrolysereaktor 6 wird z.B. mit einem Wasserdurchsatz von 60 bis 140 l/h betrieben, wobei selbstverständlich auch größere Durchsätze möglich sind, indem größere Reaktoren und/oder mehrere, parallel geschaltete Reaktoren eingesetzt werden. Vorzugsweise fährt der Elektrolysereaktor 6 stets unter Vollast, wobei er bedarfsweise abgeschaltet werden kann und Spitzenlasten über einen weiter unten noch näher erläuterten Speichertank für die elektrochemisch aktivierte, anodische, verdünnte Wasser-/Elektrolytlösung abgefangen werden können.

Wie wiederum aus Fig. 1 ersichtlich, mündet der Auslaß 66b aus dem Kathodenraum des Elektrolysereaktors 6 in einen Gasabscheider 10, aus welchem das Abgas über eine optional vorgesehene Abgasleitung 11 abgeführt wird, während der Katholyt selbst, d.h. die aus dem Kathodenraum des Elektrolysereaktors 6 abgeführte Wasser-/Elektrolytlösung, über eine Leitung 12, z.B. in die Kanalisation eines kommunalen Abwassersystems, abgeführt wird. Der Auslaß 66a aus dem Anodenraum des Elektrolysereaktors 6 mündet in einen Speichertank 13, aus welchem der Anolyt über eine Leitung 14 der Hauptwasserleitung 1 zugesetzt werden kann, was beim vorliegenden Ausführungsbeispiel über eine Bypaßleitung 15 geschieht, welche durch jeweils ein stromab bzw. stromauf der Anschlußstelle der Leitung 14 in die Bypaßleitung 15 mittels je eines Steuerventils 16, 17 auf- und zusteuerbar ist. Ein weiteres Steuerventil 18 ist in dem von der Bypaßleitung 15 überbrückten Abschnitt der Hauptwasserleitung 1 angeordnet. In der den Speichertank 13 mit der Bypaßleitung 15 der Hauptwasserleitung 1 verbindenden Leitung 14 ist ferner eine Dosierpumpe 19 vorgesehen, welche zum gesteuerten Zudosieren des Anolyts aus dem Speichertank 13 in die Hauptwasserleitung 1 dient. Eine Abgasleitung 20 mündet aus dem Speichertank 20 in die Abgasleitung 11 aus dem Gasabscheider 10. Die Funktion der Bypaßleitung 15, welcher das Desinfektionsmittel zugesetzt wird, besteht darin, daß im Normalbetrieb der gesamte, in der Hauptwasserleitung 1 geführte Wasserstrom über die Bypaßleitung 15 geführt und mit dem Desinfektionsmittel beaufschlagt werden kann. Zu Wartungs- und Installationszwecken kann die Bypaßleitung 15 indes von der Hauptwasserleitung 1 über die Ventile 16, 17 abgetrennt werden.

Der Elektrolysereaktor 6 ist des weiteren mit einer in Fig. 1 nicht näher wiedergegebenen, steuerbaren Spannungsquelle ausgestattet, um zwischen der Anode 61 und der Kathode 62 (Fig. 2) den von einem Amperemeter (nicht gezeigt) gemessenen, gewünschten Stromfluß zu steuern. Er weist ferner einen z.B. in dem Auslaß 66a für den Anolyt angeordneten pH-Meter (ebenfalls nicht gezeigt) auf, welcher alternativ beispielsweise auch in dem Speichertank 13 vorgesehen sein kann. Eine z.B. in den Reaktor 6 integrierte, steuerbare Pumpe (ebenfalls nicht gezeigt) dient zur steuerbaren Förderung der verdünnten Wasser-/Elektrolytlösung durch den Elektrolysereaktor hindurch, wobei die Pumpe den Volumenstrom und folglich die Verweilzeit der Wasser-/Elektrolytlösung in dem Reaktor 6 steuert. Eine ebenfalls nicht näher wiedergegebenen Steuereinrichtung, z.B. in Form einer elektronischen Datenverarbeitungseinheit, ist zur Steuerung der genannten Parameter derart eingerichtet, um in dem aus dem Anodenraum der Reaktors 2 über den Auslaß 66a austretenden Anolyt einen pH-Wert zwischen 2,5 und 3,5, vorzugsweise im

Bereich von etwa 3,0, aufrechtzuerhalten, was beispielsweise mittels PID-Reglern geschehen kann.

Zur Reinigung des Elektrolysereaktors 6 ist ferner ein Speicher 21 zur Aufnahme von Reinigungsflüssigkeit, z.B. Essigsäure oder dergleichen, sowie - optional - ein Speicher 22 zur Aufnahme von verbrauchter Reinigungsflüssigkeit vorgesehen, wobei eine von dem Speicher 21 in den Reaktor 6 führende Zuleitung 23 mit den Einlässen 65a, 65b des Reaktors 6 (vgl. Fig. 2) sowie eine aus dem Reaktor 6 in den Speicher 22 führende Ableitung 24 mit den Auslässen 66a, 66b des Reaktors 6 (vgl. Fig. 2) bedarfsweise koppelbar ist, um den Reaktor 6, d.h. sowohl dessen Kathodenraum als auch insbesondere dessen Anodenraum zu spülen. Alternativ kann die Reinigungslösung insbesondere im Falle von Essigsäure auch direkt in ein z.B. kommunales Abwassersystem eingespeist werden.

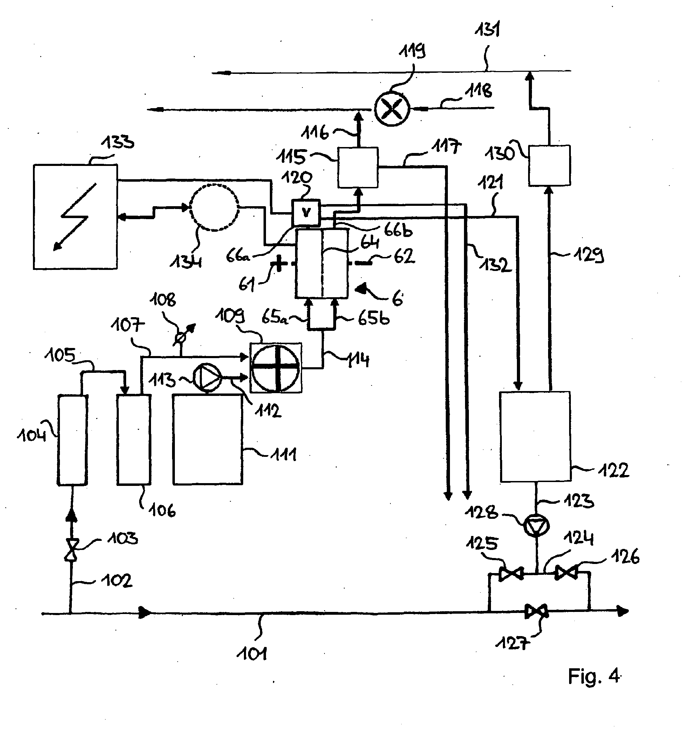

Um die Lebensdauer des Elektrolysereaktors 6 zu erhöhen bzw. um dessen Wartungsintervalle zu verlängern, kann diesem ferner ein in Fig. 1 nicht dargestellter Enthärter vorgeschaltet sein, welcher die Härte des Wassers z.B. auf einem Wert von höchstens 4° hält (vgl. in diesem Zusammenhang auch die weiter unten beschriebene Ausführungsform gemäß Fig. 4).

Nachfolgend ist die Betriebsweise der Vorrichtung zur Desinfektion von Wasser durch elektrochemische Aktivierung (ECA) unter potentialkontrollierter anodischer Oxidation (PAO) kurz erläutert.

Der Elektrolysereaktor 6 wird zunächst in Abhängigkeit der elektrischen Leitfähigkeit oder - beim vorliegenden Ausführungsbeispiel - in Abhängigkeit der hierfür etwa repräsentativen Härte des zu desinfizierenden Wassers (welches in der Regel einen pH-Wert im neutralen Bereich, d.h. von etwa 6 bis 8 aufweist) kalibriert, um zur Erzielung eines pH-Wertes von etwa 3 in dem Anodenraum des Reaktors 6 geeignete Sollwerte des zwischen den Elektroden fließenden Stromes und des Volumenstromes durch den Reaktor bzw. der Verweilzeit der Wasser-/Elektrolytlösung in dem Reaktor 6, insbesondere im Anodenraum desselben, in welchem der zur Desinfektion des Wassers wirksame Anolyt erzeugt wird, zu erhalten. Dabei gilt grundsätzlich, daß mit steigender Härte (bzw. elektrischer Leitfähigkeit) des zu desinfizierenden Wassers ein höherer Strom und/oder ein geringerer Volumenstrom (bzw. eine höhere Verweilzeit) erforderlich ist, um einen zur Einstellung eines pH-Wertes im Bereich von 3 geeigneten Umsatz der verdünnten Wasser-/Elektrolytlösung anläßlich deren elektrochemischen Aktivierung zu erzielen. Bei der Kalibrierung wird zunächst ein Volumenstrom durch den Reaktor 6 eingestellt, welcher etwa den Vorgaben des jeweils erforderlichen Volumenstromes durch den Reaktor 6 - genauer: des über die Abzweigleitung 2 dem Reaktor 6 zugeführten Volumenstromes, hier z.B. etwa 1/200 des in der Hauptwasserleitung 1 geführten Stromes an Wasser - entspricht, welcher sich vornehmlich nach der in der Hauptwasserleitung 1 geförderten Menge des mittels des über die Leitung 14 in die Hauptwasserleitung 1 zurückgeführten Anolyts (hier z.B. etwa 1/400 des in der Hauptwasserleitung 1 des in der Hauptwasserleitung 1 geführten Stromes an Wasser, während etwa 1/400 dieses Stromes in Form des Katholyts verworfen wird) zu desinfizierenden Wassers richtet. Ferner wird ein Strom eingestellt, welcher durch geeignete Zudosierung an Elektrolytlösung aus dem Reservoir 7 einen pH-Wert im Bereich von 3 der verdünnten Wasser-/Elektrolytlösung anläßlich der elektrochemischen Aktivierung ergibt. Nachdem die Kalibrierung für verschiedene Wässer mit unterschiedlicher Härte durchgeführt worden ist, werden beim vorliegenden Ausführungsbeispiel folgende Kalibriergeraden für den statischen Sollstrom (I_{soll, stat}) bzw, für den Sollvolumenstrom durch den Reaktor 6 (V'ₛₒₗₗ) erhalten:

(I) I_{soll, stat} = 0,418 A Härte [°] + 0,953 A;

(II) V'ₛₒₗₗ = -0,95 l/h · Härte [°] + 43,80 l/h.

Nachdem die vorgenannten Kalibriergeraden ermittelt worden sind, wird der zwischen der Anode 61 und der Kathode 62 des Elektrolysereaktors 6 fließende Strom je nach Härte des zu desinfizierenden Wassers auf den entsprechenden Sollwert eingestellt. Entsprechendes gilt für den Volumenstrom der verdünnten Wasser-/Elektrolytlösung durch den Reaktor 6 hindurch.

Während des Betriebs wird der pH-Wert des zur Desinfektion des Wassers verwendeten Anolyts stets so gesteuert, daß der pH-Wert des Anolyts im Bereich von 3 beträgt, was insbesondere durch zusätzliche Regelung des dynamischen Anteils (I_{soll, dyn}) des insgesamt an die Elektroden 61, 62 des Elektrolysereaktors 6 angelegten Gesamt-Sollstromes (I_{soll, ges}) unter Berücksichtigung des tatsächlich gemessenen Volumenstromes (V') durch den Reaktor 6 geschieht:

(III) I_{soll, ges} = I_{soll, stat} + I_{soll, dyn},

wobei I_{soll, dyn} = K₇ · V' + K₈. Folglich wird in Abhängigkeit des gemessenen pH-Wertes der an die Elektroden 61, 62 angelegte Strom erhöht, wenn der pH-Wert über 3 ansteigt (d.h. wenn der gemessene Volumenstrom V' zunimmt bzw. wenn der bei der elektrochemischen Aktivierung erzielte Umsatz abnimmt), während der Strom erniedrigt wird, wenn der pH-Wert unter 3 absinkt (d.h. wenn der gemessene Volumenstrom V' - z.B. in Folge einer Bildung von Gasbläschen im Reaktionsraum - abnimmt bzw. wenn der bei der elektrochemischen Aktivierung erzielte Umsatz zunimmt); und/oder der Volumenstrom V' durch den Reaktor wird verringert, wenn der pH-Wert über 3 ansteigt, während der Volumenstrom erhöht wird, wenn der pH-Wert unter 3 absinkt. Während die Menge an zudosierter Elektrolytlösung dabei vorzugsweise im wesentlichen konstant gehalten wird, kann alternativ oder zusätzlich auch vorgesehen sein, daß mehr Elektrolytlösung aus dem Reservoir 7 zudosiert wird, wenn der pH-Wert über 3 ansteigt (d.h. wenn der bei der elektrochemischen Aktivierung erzielte Umsatz abnimmt), während weniger Elektrolytlösung zudosiert wird, wenn der pH-Wert unter 3 absinkt (d.h. wenn der bei der elektrochemischen Aktivierung erzielte Umsatz ansteigt). Insbesondere ist es erfindungsgemäß auch möglich, zwei der drei vorgenannten Parameter Strom (d.h. elektrischer Strom zwischen den Elektroden des Reaktors 6), Volumenstrom durch den Reaktor 6 hindurch (d.h. Volumenstrom der verdünnten Wasser-/Elektrolytlösung) und Menge an zudosierter Elektrolytlösung konstant zu halten und den pH-Wert lediglich durch Steuerung des dritten Parameters in dem erfindungsgemäßen Bereich zu halten. Das Redoxpotential, welches sich bei der erfindungsgemäßen Steuerung des pH-Wertes auf einen Wert im Bereich von 3 einstellt, beträgt vorzugsweise etwa konstant 1340 mV ± 20 mV.

Die auf diese Weise durch die erfindungsgemäß gesteuerte elektrochemische Aktivierung in Form einer potentialkontrollierten anodischen Oxidation erhaltene Desinfektionsflüssigkeit, welche in Form des Anolyts in dem Speichertank 13 zwischengespeichert wird, wird der Hauptwasserleitung 1 mittels der Dosierpumpe 19 - insbesondere in einem Anteil von etwa 1:400 - zugesetzt, um für eine zuverlässige Desinfektion des gesamten, hierin geförderten Wassers zu sorgen. Da sich der elektrochemisch aktivierte Anolyt, wie bereits erwähnt, in einem metastabilen Zustand befindet, sollte er im Hinblick auf die weitestgehend ungeschützte und gegebenenfalls warme Lagerung mit relativ großer freier Oberfläche des Flüssigkeitsspiegels in dem Vorratstank 13 maximal etwa 14 Tage, vorzugsweise maximal etwa 48 Stunden, in dem Speichertank 13 auf Vorrat gehalten werden, bevor er dem Wasser zum Zwecke der Desinfektion zugesetzt wird. Indes besteht, wie weiter oben erwähnt, ohne weiteres die Möglichkeit, das auf die vorgenannten Weise herstellte Desinfektionsmittel selbst bis zu etwa sechs Monate zu lagern, wobei für einen möglichst gasdichten Abschluß entsprechender Lagertanks und vorzugsweise auch für eine möglichst niedrige Temperatur, z.B. bis zu etwa 8°C, gesorgt werden sollte.

Fig. 4 zeigt ein schematisches Verfahrensschema einer weiteren Vorrichtung zur kontinuierlichen oder semikontinuierlichen Durchführung eines erfindungsgemäßen Verfahrens zur Desinfektion von Wasser durch elektrochemische Aktivierung (ECA). Die Vorrichtung umfaßt wiederum eine Hauptwasserleitung 101, in welcher das zu desinfizierende Wasser gefördert wird, z.B. in Form einer Zulaufleitung der Wasserversorgung eines Krankenhauses, eines Gewerbebetriebs, eines Hotels oder eines anderen gastronomischen Betriebs, von der Umwälzleitung eines Swimmingpools oder der gleichen. An die Hauptwasserleitung 101 ist eine Abzweigleitung 102 angeschlossen, welche mit einem Ventil 103, wie einem Steuerventils, ausgestattet ist und darüber hinaus ein Filter (nicht dargestellt), insbesondere in Form eines Feinfilters aufweisen kann.

Die Abzweigleitung 103 mündet stromab des Ventils 103 in einen Enthärter 104, welcher z.B. mit einem geeigneten Ionenaustauscherharz bestückt ist und die in dem Wasser enthaltenen zweiwertigen Härtebildner Calcium- und Magnesiumionen durch einwertige Ionen, wie beispielsweise Natrium, ersetzt. Um die Lebensdauer des Elektrolysereaktors 6 zu erhöhen bzw. um dessen Wartungsintervalle zu verlängern, hält der Enthärter 104 die Härte des Wassers z.B. auf einem Wert von höchstens 4° dH (entsprechend einer Konzentration an Erdalkalimetallionen von 0,716 mMol/l), vorzugsweise von höchstens 2° dH (entsprechend einer Konzentration an Erdalkalimetallionen von 0,358 mMol/l). Der Ablauf 105 des Enthärters 104 mündet in eine Einrichtung 106 zur Verminderung der spezifischen elektrischen - bzw. ionischen - Leitfähigkeit des Wassers, welche insbesondere von einer Membrananlage, wie einer Umkehrosmoseanlage oder von einer Mikro-, Nano- öder Ultrafiltrationsanlage gebildet sein kann und die spezifische elektrische Leitfähigkeit des Wassers auf einem Wert von höchstens 350 µS/cm, insbesondere von höchstens 150 µS/cm, vorzugsweise von höchstens 100 µS/cm, hält. Im Ablauf 107 der Membrananlage 106 ist eine Leitfähigkeitsmeßeinrichtung 108, wie eine Leitfähigkeitsmeßzelle, -elektrode oder dergleichen, zur Überwachung der Einhaltung des jeweils gewünschten Wertes der spezifischen elektrischen Leitfähigkeit des Wassers angeordnet.

Darüber hinaus können insbesondere dann, wenn das zu desinfizierende Wasser einen verhältnismäßig hohen organischen Kohlenstoffgehalt aufweist, Maßnahmen vorgesehen sein, um den Kohlenstoffgehalt des Wassers zu erniedrigen. Hierzu kann beispielsweise eine dem Mischer 109 vorgeschaltete UV-Oxidationsanlage (nicht gezeigt) vorgesehen sein, welche den gesamten organischen Kohlenstoffgehalt (TOC) und/oder den chemischen Sauerstoffbedarf (COD) auf einen Wert von höchstens 25 ppb, insbesondere von höchstens 20 ppb, bzw. auf einen Wert von höchstens 7 mg O₂/l, insbesondere von höchstens 5 mg O₂/l, absenkt. Zur Messung und/oder Kontrolle des TOC- bzw. COD-Wertes - oder auch anderer Gruppenparameter zur Erfassung des im Wasser enthaltenen organischen Kohlenstoffes, wie des gelösten organischen Kohlenstoffes (DOC, dissolved organic carbon) - können aus dem Stand der Technik bekannte Einrichtungen vorgesehen sein. Ebenfalls ist es auch in Verbindung mit dem Enthärter 104 und der Membrananlage 106 denkbar, den über die Abzweigleitung 102 aus der Hauptwasserleitung 101 abgezweigten, elektrochemisch zu aktivierenden Teilstrom des Wassers nur bedarfsweise durch den Enthärter 104, die Membrananlage 106 bzw. die UV-Oxidationsanlage zu führen, nämlich dann, wenn der jeweilige Grenzwert überschritten wird, und die jeweilige Anlage andernfalls mittels einer Bypaßleitung (nicht gezeigt) zu überbrücken.

Der Ablauf 107 der Membrananlage 106 führt in einen Mischer 109, welcher in einen Elektrolysereaktor 6 mündet. Letzterer entspricht im vorliegenden Fall dem in Fig. 2 wiedergegebenen Ausführungsbeispiel. Über die Abzweigleitung 102 ist somit ein mittels des Steuerventils 103 steuerbarer, enthärteter und deionisierter Teilstrom des in der Hauptwasserleitung 101 geförderten Wassers in den Elektrolysereaktor 6 überführbar, wobei z.B. ein Teilstrom des in der Hauptwasserleitung 101 geförderten Wassers in der Größenordnung von 1/200 über die Abzweigleitung 102 abgezweigt wird. Der Mischer 109 steht zulaufseitig einerseits - wie bereits erwähnt - mit dem Ablauf 107 der Membrananlage 106, andererseits mit einem Reservoir 111 zur Aufnahme einer Elektrolytlösung, insbesondere in Form einer im wesentlichen gesättigten Alkalimetallchloridlösung - im vorliegenden Fall einer Natriumchloridlösung -, in Verbindung, welche in dem Mischer 109 möglichst homogen miteinander vermischt werden und über eine gemeinsame, ablaufseitige Leitung 114 des Mischers 109 in den Elektrolysereaktor 6 gelangen. Die von dem Reservoir 111 in den Mischer 109 führende Leitung 112 ist ferner mit einer Dosierpumpe 113 ausgestattet, um dem elektrochemisch zu aktivierenden Wasser eine definierte Menge an Natriumchloridlösung zuzusetzen. Der Mischer 109 kann beispielsweise von einem Kugelmischer gemäß Fig. 3 gebildet sein. Der Elektrolysereaktor 6 wird wiederum beispielsweise mit einem Wasserdurchsatz von 60 bis 140 l/h betrieben, wobei er aus den oben unter Bezugnahme auf Fig. 1 genannten Gründen vorzugsweise stets unter Vollast gefahren wird und bedarfsweise abgeschaltet werden kann.

Wie weiterhin aus Fig. 4 ersichtlich, mündet der Auslaß 66b aus dem Kathodenraum des Elektrolysereaktors 6 in einen Gasabscheider 115, aus welchem das Abgas, insbesondere Wasserstoff (H₂), über eine optional vorgesehene Abgasleitung 116 abgeführt wird, während der Katholyt selbst, d.h. die aus dem Kathodenraum des Elektrolysereaktors 6 abgeführte, verdünnte Wasser-/Elektrolytlösung, über eine Leitung 117 z.B. in die Kanalisation eines kommunalen Abwassersystems abgeführt wird. Die Abgasleitung 116 mündet beim vorliegenden Ausführungsbeispiel in eine mit Verdünnungsluft gespeiste Abluftleitung 118, welche mit einem explosionsgeschützten Niederdruckgebläse 119 ausgestattet ist.

Der Auslaß 66a aus dem Anodenraum des Elektrolysereaktors 6 mündet über ein Steuerventil 120 und eine Leitung 121 in einen Speichertank 122, aus welchem der Anolyt über eine Leitung 123 der Hauptwasserleitung 101 zugesetzt werden kann. Dies geschieht beim vorliegenden Ausführungsbeispiel über eine Bypaßleitung 124, welche durch jeweils ein stromab bzw. stromauf der Anschlußstelle der Leitung 123 in die Bypaßleitung 124 mittels je eines Steuerventils 125, 126 auf- und zusteuerbar ist. Ein weiteres Steuerventil 127 ist in dem von der Bypaßleitung 124 überbrückten Abschnitt der Hauptwasserleitung 101 angeordnet. In der den Speichertank 122 mit der Bypaßleitung 124 der Hauptwasserleitung 101 verbindenden Leitung 123 ist ferner eine Dosierpumpe 128 vorgesehen, welche zum gesteuerten Zudosieren des Anolyts aus dem Speichertank 122 in die Hauptwasserleitung 101 dient. Eine Abgasleitung 129, insbesondere für in dem Anolyt gegebenenfalls freigesetztes Chlorgas (Cl₂), mündet aus dem Gasraum des Speichertanks 122 in einen Gasabscheider 130, dessen Gasraum wiederum mit einer Abführleitung für Chlorgas verbunden ist. In dem Gasabscheider abgeschiedene Flüssigkeiten, wie beispielsweise auskondensiertes Wasser, können ebenfalls z.B. in die Kanalisation eines kommunalen Abwassersystems abgeführt werden (nicht dargestellt). Die Funktion der Bypaßleitung 124, welcher das Desinfektionsmittel in Form des Anolyts, also der im Anodenraum des Elektrolysereaktors 6 elektrochemisch aktivierten, verdünnten Wasser-/Natriumchloridlösung, zugesetzt wird, besteht darin, daß im Normalbetrieb der gesamte, in der Hauptwasserleitung 101 geführte Wasserstrom über die Bypaßleitung 124 geführt und mit dem Desinfektionsmittel beaufschlagt werden kann. Zu Wartungs- und Installationszwecken kann die Bypaßleitung 124 indes von der Hauptwasserleitung 1 über die Ventile 125, 126 abgetrennt werden.

Ferner kann vorgesehen sein, daß beim Anfahren des Elektrolysereaktors 110 über einen bestimmten Zeitraum auch der "Anolyt", d.h. die elektrochemisch aktivierte, anodische Wasser-/Elektrolytlösung, verworfen wird, um anfängliche Qualitätsbeeinträchtigungen, so lange der Elektrolysereaktor 110 noch nicht seinen gewünschten Betriebszustand erreicht hat, auszuschließen. Zu diesem Zweck geht von dem Ventil 120 parallel zu der in den Speichertank 122 führenden Leitung 121 eine weitere Leitung 132 aus, welche z.B. in die Kanalisation eines kommunalen Abwassersystems führt, um - je nach Schaltstellung des Ventils 120 - auch den Anolyt verwerfen zu können.

Die Vorrichtung gemäß Fig. 4 umfaßt des weiteren eine Steuerung 133, z.B. in Form einer elektronischen Datenverarbeitungseinheit, welche einerseits mit dem Steuerventil 120, um bedarfsweise die Leitung 121 bzw. die Leitung 132 auf- und zu zu steuern, andererseits über eine Potentialkontrolle 134 mit dem Elektrolysereaktor 6 in Verbindung steht, um in dem Elektrolysereaktor 6 zwischen der Anode 61 und der Kathode 62 (Fig. 2) den z.B. von einem Amperemeter (nicht gezeigt) gemessenen, gewünschten Stromfluß zu steuern. Dies geschieht aus den eingangs erwähnten Gründen derart, daß sich zum einen in der elektrochemisch aktivierten, anodischen, verdünnten Wasser-/Elektrolytlösung ein pH-Wert im Bereich von etwa 3 einstellt und sich zum anderen ein Redoxpotential im Bereich von etwa 1340 mV ergibt, was durch die erfindungsgemäße Einstellung einer spezifischen elektrischen Leitfähigkeit des Rohwassers von höchstens 350 µS/cm auf einfache Weise für praktisch alle Wässer mit beliebigen Inhaltsstoffen möglich ist. Zu diesem Zweck ist in der aus dem Anodenraum des Elektrolysereaktors 6 führenden Leitung 66a zweckmäßig ein pH-Meter (nicht gezeigt) sowie vorzugsweise auch eine weitere Leitfähigkeitsmeßzelle oder -elektrode (ebenfalls nicht gezeigt) vorgesehen, welche es der Steuerung 133 ermöglichen, die dem Rohwasser über die Pumpe 113 zugesetzte Menge an Natriumchloridlösung derart zu steuern, daß sich die gewünschten Prozeßparameter ergeben. Die Gesamtkonzentration an Natriumchlorid im Zulauf 114 des Elektrolysereaktors 6 sollte indes etwa 20 g/l, vorzugsweise etwa 10 g/l, nicht überschreiten. Die Steuerung 133 kann ferner z.B. eine in den Reaktor 6 integrierte, steuerbare Pumpe (nicht gezeigt) zur steuerbaren Förderung der Wasser-/Elektrolytlösung durch den Elektrolysereaktor 6 hindurch steuern, wobei mittels der Pumpe folglich der Volumenstrom bzw. die Verweilzeit der verdünnten Wasser-/Elektrolytlösung durch den bzw. in dem Reaktor 6 einstellbar ist.

Nachfolgend ist die Betriebsweise der Vorrichtung zur Desinfektion von Wasser durch elektrochemische Aktivierung (ECA) unter potentialkontrollierter anodischer Oxidation (PAO) kurz erläutert.

Das über die Abzweigleitung 102 aus der Hauptwasserleitung 101 abgezweigte Wasser - z.B. ein Volumenanteil von etwa 1:200 des in der Hauptwasserleitung 101 geförderten Wassers - wird zunächst in dem Enthärter 104, z.B. auf eine Härte im Bereich von etwa 1° dH (entsprechend einer Konzentration an Erdalkalimetallionen von Calcium und Magnesium von 0,179 mmol), enthärtet, wonach in der Membrananlage 106 seine spezifische elektrische Leitfähigkeit auf einen Wert von beispielsweise etwa 50 µS/cm abgesenkt wird. Im Falle eines relativ hohen organischen Kohlenstoffgehaltes des Wassers, z.B. größer etwa 25 ppb, kann dieser ferner, z.B. durch oxidativen Abbau, vermindert werden.

Mit dem derart enthärteten und deionisierten sowie gegebenenfalls oxidativ behandelten Wasser wird eine Kalibrierung erstellt, um eine Korrelation zwischen der Menge der dem Wasser mittels der Dosierpumpe 113 zuzusetzenden Menge an Natriumchloridlösung und der erhaltenen gesamten spezifischen elektrischen Leitfähigkeit der hierdurch erzeugten, verdünnten Wasser-/Natriumchloridlösung zu erhalten. Diese Abhängigkeit der Natriumchloridkonzentration von der spezifischen elektrischen Leitfähigkeit der dem Elektrolysereaktor zugesetzten, verdünnten Wasser-/Natriumchloridlösung wird dabei insbesondere gemäß einer Kalibriergeraden der Form

κ_{ges} = κ_{w} + dκ/d[NaCl) · [NaCl]

ermittelt, wobei κ_{ges} die spezifische elektrische Leitfähigkeit der dem Elektrolysereaktor zugesetzten, verdünnten Wasser-/Elektrolytlösung, κ_{w} die spezifische Leitfähigkeit des eingesetzten, zu desinfizierenden Wassers (unmittelbar vor dem Zusetzen der Elektrolytlösung, d.h. im vorliegenden Fall mit einer spezifischen elektrischen Leitfähigkeit von etwa 50 µS/cm), [NaCl] die Natriumchloridkonzentration der eingesetzten, verdünnten Wasser-/Natriumchloridlösung und dκ/d[NaCl] die wasserspezifische Steigung der Kalibriergeraden ist.

Sodann kann der Elektrolysereaktor 6 für das zu desinfizierende Wasser kalibriert werden, um zur Erzielung eines pH-Wertes von etwa 3 in dem Anodenraum des Reaktors 6 geeignete Sollwerte des zwischen den Elektroden 61, 62 fließenden Stromes und des Volumenstromes durch den Reaktor 6 bzw. der Verweilzeit der verdünnten Wasser-/Elektrolytlösung in dem Reaktor 6, insbesondere im Anodenraum desselben, in welchem der zur Desinfektion des Wassers wirksame Anolyt erzeugt wird, zu erhalten. Dabei gilt grundsätzlich, daß mit steigender elektrischer Leitfähigkeit des zu desinfizierenden Wassers ein höherer Strom und/oder ein geringerer Volumenstrom (bzw. eine höhere Verweilzeit) erforderlich ist, um einen zur Einstellung eines pH-Wertes im Bereich von 3 geeigneten Umsatz der verdünnten Wasser-/Elektrolytlösung anläßlich deren elektrochemischen Aktivierung zu erzielen.

Während des Betriebs wird der pH-Wert des zur Desinfektion des Wassers verwendeten Anolyts stets so gesteuert, daß der pH-Wert des Anolyts im Bereich von 3 beträgt, was insbesondere durch entsprechende Regelung des an die Elektroden 61, 62 des Elektrolysereaktors 6 angelegten elektrischen Stromes unter Berücksichtigung des Volumenstromes der Wasser-/Natriumchloridlösung durch den Reaktor 6 und/oder durch entsprechende Zudosierung des Natriumchloridlösung mittels der Dosierpumpe 113 geschieht. Das Redoxpotential, welches sich bei einer solchen Steuerung des pH-Wertes auf einen Wert im Bereich von 3 einstellt, beträgt vorzugsweise etwa konstant 1340 mV ± 20 mV vs. SHE.

Die auf diese Weise durch die erfindungsgemäß gesteuerte elektrochemische Aktivierung in Form einer potentialkontrollierten anodischen Oxidation erhaltene Desinfektionsflüssigkeit, welche in Form des Anolyts in dem Speichertank 122 zwischengespeichert wird, wird der Hauptwasserleitung 101 mittels der Dosierpumpe 128 - z.B. in einem Anteil von etwa 1:400 - zugesetzt, um für eine zuverlässige Desinfektion des gesamten, hierin geförderten Wassers zu sorgen. Der Katolyt kann über die Leitung 132 verworfen werden.

### Vergleichsbeispiel:

Herstellung einer elektrochemisch aktivierten, anodischen, verdünnten Wasser-/Elektrolytlösung ("Anolyt") mittels einer Vorrichtung gemäß Fig. 4 (A) im Vergleich mit der Herstellung eines Anolyts mit derselben Vorrichtung, aber unter Überbrückung der Umkehrosmoseanlage 106 zur Absenkung der Leitfähigkeit des eingesetzten Wassers sowie des Ionentauschers 104 (B).

### Eingesetztes Rohwasser:

Elektrische Leitfähigkeit (κ_{W}): 543 µS/cm;
Gesamthärte: 14,3°dH (hiervon 10,7°dH Carbonat);
pH-Wert: 7,63.

### Rohwasser nach Absenkung der elektrischen Leitfähigkeit (A):

Elektrische Leitfähigkeit (κ_{W}) : 89 µS/cm;
pH-Wert: 7,25.

### Anolyt aus (A):

Elektrische Leitfähigkeit: 9820 pS/cm;
Freies Chlor: 50,6 mg/l (schwankend);
Gesamtchlor: 56,6 mg/l (schwankend);
gebundenes Chlor: 6,00 mg/l (schwankend);
pH-Wert: 4,00 (schwankend).

### Anolyt aus (B):

Elektrische Leitfähigkeit: 3950 µS/cm;
Freies Chlor: 19,9 mg/l;
Gesamtchlor: 19,9 mg/l;
gebundenes Chlor: < 0,05 mg/l;
pH-Wert: 3,10.

Das Experiment zeigt, daß sich bei dem vorliegenden Rohwasser mit einer spezifischen elektrischen Leitfähigkeit von etwa 550 µS/cm und einer Härte von 14,3°dH (entsprechend 2,560 mMol/l Erdalkalimetallionen) ohne die Absenkung der elektrischen Leitfähigkeit ein pH-Wert um 3 in der Praxis auch bei relativ hohen Mengen an zudosierter Natriumchloridlösung nicht exakt einstellen läßt. Entsprechendes gilt für das erwünschte Redoxpotential im Bereich von 1340 mV vs. SHE. Im Gegensatz zu der Vorgehensweise (A) ist die Reproduzierbarkeit im Falle von (B) schlechter und sind die Chlorwerte so stark erhöht, daß die Gefahr besteht, daß sie nicht mehr der deutschen Trinkwasserverordnung (TrinkwV) genügen. Die Desinfektionswirkung des Anolyts ist somit nicht mit absoluter Sicherheit vorhersagbar.

## Patentansprüche

1. Verfahren zur Herstellung eines Desinfektionsmittels durch elektrochemische Aktivierung (ECA) von Wasser, indem dem Wasser eine Elektrolytlösung, insbesondere eine Natrium- und/oder Kaliumchloridlösung, zugesetzt und das mit der Elektrolytlösung beaufschlagte Wasser in Form einer verdünnten Wasser-/Elektrolytlösung in einem Elektrolysereaktor (6) mit wenigstens einem Kathodenraum mit einer Kathode (62) und mit wenigstens einem von dem Kathodenraum räumlich, insbesondere mittels eines Diaphragmas oder einer Membran, getrennten Anodenraum mit einer Anode (61) durch Anlegen einer Gleichspannung an die Elektroden (61, 62) mit einem elektrischen Strom beaufschlagt wird, um die verdünnte Wasser-/Elektrolytlösung in einen zur Desinfektion geeigneten, metastabilen Zustand zu versetzen, wobei der pH-Wert der verdünnten Wasser-/Elektrolytlösung in dem Anodenraum auf einen Wert zwischen 2,7 und 3,5 und das Redoxpotential der verdünnten Wasser-/Elektrolytlösung in dem Anodenraum auf einen Wert zwischen 1240 mV und 1360 mV bezogen auf die Standardwasserstoffelektrode (SHE) gesteuert wird, indem
(a) bei einer vorgegebenen Verweilzeit (T_{V}) der Wasser-/Elektrolytlösung in dem Elektrolysereaktor (6) und/oder bei einem vorgegebenen Volumenstrom (V') der Wasser-/Elektrolytlösung durch den Elektrolysereaktor (6) ein von der jeweiligen Zusammensetzung des zu desinfizierenden Wassers abhängiger, statischer Sollstrom (I_{soll, stat}) zwischen den Elektroden (61, 62) in Abhängigkeit von der Leitfähigkeit (κ) und/oder von der Härte (H) des Wassers gemäß einer Geradengleichung der Form
I_{soll, stat} = K₁ ˙ κ + K₂
und/oder
I_{soll, stat} = K₃ ˙ H + K₄
ermittelt wird, wobei K₁, K₂ ,K₃ und K₄ reaktorspezifische Konstanten sind; und
(b) bei einem vorgegebenen statischen Sollstrom (I_{soll, stat}) zwischen den Elektroden (61, 62) des Elektrolysereaktors (6) ein von der jeweiligen Verweilzeit (T_{V}) der Wasser-/Elektrolytlösung in dem Elektrolysereaktor und/ oder von dem jeweiligen Volumenstrom der Wasser-/Elektrolytlösung durch den Elektrolysereaktor (6) abhängiger, dynamischer Sollstrom (I_{soll, dyn}) gemäß einer Geradengleichung der Form
I_{soll, dyn} = K₅ ˙ T_{V} + K₆
und/oder
I_{soll, dyn} = K₇ ˙ V' + K₈
ermittelt wird, wobei K₅, K₆, K₇ und K₈ reaktorspezifische Konstanten sind; und
(c) an die Elektroden (61, 62) des Elektrolysereaktors (6) ein Gesamt-Sollstrom (I_{soll, ges}) angelegt wird, welcher aus der Summe des statischen Sollstromes (I_{soll, stat}) und des dynamischen Sollstromes (I_{soll, dyn}) gebildet ist:
I_{soll, ges} = I_{soll, stat} + I_{soll, dyn}.

2. Verfahren nach Anspruch 1, **dadurch gekennzeichnet, daß** der pH-Wert der verdünnten Wasser-/Elektrolytlösung in dem Anodenraum auf einen Wert zwischen 2,7 und 3,3, insbesondere auf einen Wert zwischen 2,8 und 3,2, gesteuert wird.

3. Verfahren nach Anspruch 1 oder 2, **dadurch gekennzeichnet, daß** das Redoxpotential der verdünnten Wasser-/Elektrolytlösung in dem Anodenraum auf einen Wert zwischen 1280 mV und 1360 mV bezogen auf die SHE, insbesondere auf einen Wert zwischen 1320 mV und 1360 mV bezogen auf die SHE, gesteuert wird.

4. Verfahren nach einem der Ansprüche 1 bis 3, **dadurch gekennzeichnet, daß** der pH-Wert der verdünnten Wasser-/Elektrolytlösung in dem Anodenraum durch gesteuertes Zusetzen der entsprechenden Menge an Elektrolytlösung gesteuert wird.

5. Verfahren nach einem der Ansprüche 1 bis 4, **dadurch gekennzeichnet, daß** der pH-Wert der verdünnten Wasser-/Elektrolytlösung in dem Anodenraum durch Steuerung der Verweilzeit (Tv) und/oder des Volumenstromes (V') derselben in dem bzw. durch den Elektrolysereaktor (6) gesteuert wird.

6. Verfahren nach einem der Ansprüche 1 bis 5, **dadurch gekennzeichnet, daß** zur Steuerung des pH-Wertes der verdünnten Wasser-/Elektrolytlösung in dem Anodenraum des Elektrolysereaktors (6) auf einen pH-Wert zwischen 2,7 und 3,5 bei einem vorgegebenen Sollstrom zwischen den Elektroden (61, 62) des Elektrolysereaktors (6) eine von der jeweiligen Zusammensetzung des zu desinfizierenden Wassers abhängige Verweilzeit (T_{V}) und/oder eine von der jeweiligen Zusammensetzung des Wassers abhängiger Volumenstrom (V') der verdünnten Wasser-/Elektrolytlösung in dem bzw. durch den Elektrolysereaktor (6) ermittelt wird.

7. Verfahren nach Anspruch 6, **dadurch gekennzeichnet, daß** die Verweilzeit (T_{V}) und/oder der Volumenstrom (V') in dem bzw. durch den Elektrolysereaktor (6) in Abhängigkeit von der Leitfähigkeit (κ) und/oder von der Härte (H) des Wassers ermittelt wird.

8. Verfahren nach Anspruch 6 oder 7, **dadurch gekennzeichnet, daß** die Verweilzeit (T_{V}) und/oder der Volumenstrom (V') gemäß einer Geradengleichung der Form
T_{V} = k₁ ˙ κ + k₂
und/oder
V' = k₃ ˙ H + k₄
ermittelt wird, wobei k₁, k₂ ,k₃ und k₄ reaktorspezifische Konstanten sind.

9. Verfahren nach einem der Ansprüche 6 bis 8, **dadurch gekennzeichnet, daß** die Menge an zudosierter Elektrolytlösung im wesentlichen konstant gehalten wird.

10. Verfahren nach einem der Ansprüche 1 bis 9, **dadurch gekennzeichnet, daß** die Elektrolytkonzentration der dem Elektrolysereaktor (10) zugesetzten, verdünnten Wasser-/Elektrolytlösung auf einen Wert von höchstens 20 g/l gesteuert wird.

11. Verfahren nach einem der Ansprüche 1 bis 10, **dadurch gekennzeichnet, daß** die spezifische elektrische Leitfähigkeit des elektrochemisch zu aktivierenden Wassers vor Zusetzen der Elektrolytlösung auf einen Wert von höchstens 350 µS/cm eingestellt wird.

12. Verfahren nach einem der Ansprüche 1 bis 11, **dadurch gekennzeichnet, daß** die Steuerung der Elektrolytkonzentration, insbesondere der Alkalimetallchloridkonzentration, der dem Elektrolysereaktor (10) zugesetzten, verdünnten Wasser-/Elektrolytlösung, insbesondere der verdünnten Wasser-/Alkalimetallchloridlösung, durch Steuerung der dem elektrochemisch zu aktivierenden Wasser zugesetzten Menge an Elektrolytlösung geschieht.

13. Verfahren nach Anspruch 12, **dadurch gekennzeichnet, daß** die Steuerung der Alkalimetallchloridkonzentration der dem Elektrolysereaktor (10) zugesetzten, verdünnten Wasser-/Alkalimetallchloridlösung in Abhängigkeit der hiermit korrespondierenden spezifischen elektrischen Leitfähigkeit der dem Elektrolysereaktor (10) zugesetzten Wasser-/Alkalimetallchloridlösung durchgeführt wird, wobei die Abhängigkeit der Alkalimetallchloridkonzentration von der spezifischen elektrischen Leitfähigkeit der dem Elektrolysereaktor (10) zugesetzten, verdünnten Wasser-/Alkalimetallchloridlösung für das jeweils eingesetzte, elektrochemisch zu aktivierende Wasser vorab ermittelt wird.

14. Verfahren nach Anspruch 13, **dadurch gekennzeichnet, daß** die Abhängigkeit der Alkalimetallchloridkonzentration von der spezifischen elektrischen Leitfähigkeit der dem Elektrolysereaktor (10) zugesetzten, verdünnten Wasser-/Alkalimetallchloridlösung gemäß einer Kalibriergeraden der Form
κ_{ges} = κ_{w} + dκ/d[MeCl] ˙ [MeCl]
ermittelt wird, wobei κ_{ges} die spezifische elektrische Leitfähigkeit der dem Elektrolysereaktor (10) zugesetzten, verdünnten Wasser-/Alkalimetallchloridlösung, κ_{w} die spezifische elektrische Leitfähigkeit des jeweils eingesetzten, zu desinfizierenden Wassers, [MeCl] die Alkalimetallchloridkonzentration der verdünnten Wasser-/Alkalimetallchloridlösung und dκ/d[MeCl] die wasserspezifische Steigung der Kalibriergeraden ist.

15. Verfahren nach einem der Ansprüche 1 bis 14, **dadurch gekennzeichnet, daß** ausschließlich die in dem Anodenraum erzeugte, elektrochemisch aktivierte, verdünnte Wasser-/Elektrolytlösung als Desinfektionsmittel verwendet wird.

## Claims

1. A method for the production of a disinfectant by electrochemical activation (ECA) of water, by adding an electrolyte solution, in particular a sodium chloride and/or potassium chloride solution, to the water and exposing the water, to which the electrolyte solution has been added, in the form of a dilute water/electrolyte solution to an electric current in an electrolysis reactor (6) with at least one cathode compartment with a cathode (62) and with at least one anode compartment with an anode (61), which anode compartment is spatially separated from the cathode compartment, in particular by means of a diaphragm or a membrane, by application of a direct voltage to the electrodes (61, 62) in order to convert the dilute water/electrolyte solution into a metastable state suitable for disinfection, the pH value of the dilute water/electrolyte solution in the anode compartment being controlled to a value of between 2.7 and 3.5 and the redox potential of the dilute water/electrolyte solution in the anode compartment to a value of between 1240 mV and 1360 mV relative to the standard hydrogen electrode (SHE), by
(a) a static nominal current (I_{nominal, stat}), dependent on the particular composition of the water to be disinfected, being determined between the electrodes (61, 62) at a predetermined residence time (Tᵥ) of the water/electrolyte solution in the electrolysis reactor (6) and/or at a predetermined volumetric flow rate (V) of the water/electrolyte solution through the electrolysis reactor (6), as a function of the conductivity (κ) and/or of the hardness (H) of the water according to a linear equation of the form
I_{nominal, stat} = K₁ · κ + K₂
and/or
I_{nominal, stat} = K₃ · H + K₄
K₁, K₂, K₃ and K₄ being reactor-specific constants; and
(b) a dynamic nominal current (I_{nominal, dyn}), dependent on the particular residence time (Tᵥ) of the water/electrolyte solution in the electrolysis reactor and/ or on the particular volumetric flow rate of the water/electrolyte solution through the electrolysis reactor (6), being determined at a predetermined static nominal current (I_{nominal, stat}) between the electrodes (61, 62) of the electrolysis reactor (6), according to a linear equation of the form
I_{nominal, dyn} = K₅ · Tᵥ + K₆
and/or
I_{nominal, dyn} = K₇ · V' + K₈
K₅, K₆, K₇ and K₈ being reactor-specific constants; and
(c) a total nominal current (I_{nominal, total}) being applied to the electrodes (61, 62) of the electrolysis reactor (6), which total nominal current is formed from the sum of the static nominal current (I_{nominal, stat}) and the dynamic nominal current (I_{nominal, dyn}):
I_{nominal, total} = I_{nominal, stat} + I_{nominal, dyn}.

2. A method according to claim 1, **characterised in that** the pH value of the dilute water/electrolyte solution in the anode compartment is controlled to a value of between 2.7 and 3.3, in particular to a value of between 2.8 and 3.2.

3. A method according to claim 1 or claim 2, **characterised in that** the redox potential of the dilute water/electrolyte solution in the anode compartment is controlled to a value of between 1280 mV and 1360 mV relative to the SHE, in particular to a value of between 1320 mV and 1360 mV relative to the SHE.

4. A method according to any one of claims 1 to 3, **characterised in that** the pH value of the dilute water/electrolyte solution in the anode compartment is controlled by controlled addition of the corresponding quantity of electrolyte solution.

5. A method according to any one of claims 1 to 4, **characterised in that** the pH value of the dilute water/electrolyte solution in the anode compartment is controlled by controlling the residence time (Tᵥ) and/or the volumetric flow rate (V') thereof in or through the electrolysis reactor (6).

6. A method according to any one of claims 1 to 5, **characterised in that** the pH value of the dilute water/electrolyte solution in the anode compartment of the electrolysis reactor (6) is controlled to a pH value of between 2.7 and 3.5 at a predetermined nominal current between the electrodes (61, 62) of the electrolysis reactor (6) by determining a residence time (Tᵥ) dependent on the particular composition of the water to be disinfected and/or a volumetric flow rate (V') of the dilute water/electrolyte solution in or through the electrolysis reactor (6) dependent on the particular composition of the water.

7. A method according to claim 6, **characterised in that** the residence time (Tᵥ) and/or the volumetric flow rate (V) in or through the electrolysis reactor (6) is determined as a function of the conductivity (κ) and/or the hardness (H) of the water.

8. A method according to claim 6 or claim 7, **characterised in that** the residence time (Tᵥ) and/or the volumetric flow rate (V) is determined according to a linear equation of the form
Tᵥ = k₁ · κ + k₂
and/or
V' = k₃ · H + k₄
k₁, k₂, k₃ and k₄ being reactor-specific constants.

9. A method according to any one of claims 6 to 8, **characterised in that** the quantity of electrolyte solution apportioned is kept substantially constant.

10. A method according to any one of claims 1 to 9, **characterised in that** the electrolyte concentration of the dilute water/electrolyte solution added to the electrolysis reactor (10) is controlled to a value of at most 20 g/I.

11. A method according to any one of claims 1 to 10, **characterised in that** the specific electrical conductivity of the water to be electrochemically activated is adjusted to a value of at most 350 µS/cm before addition of the electrolyte solution.

12. A method according to any one of claims 1 to 11, **characterised in that** control of the electrolyte concentration, in particular of the alkali metal chloride concentration, of the dilute water/electrolyte solution, in particular the dilute water/alkali metal chloride solution, added to the electrolysis reactor (10) is effected by controlling the quantity of electrolyte solution added to the water to be electrochemically activated.

13. A method according to claim 12, **characterised in that** the alkali metal chloride concentration of the dilute water/alkali metal chloride solution added to the electrolysis reactor (10) is controlled as a function of the specific electrical conductivity, corresponding thereto, of the water/alkali metal chloride solution added to the electrolysis reactor (10), the dependency of the alkali metal chloride concentration on the specific electrical conductivity of the dilute water/alkali metal chloride solution added to the electrolysis reactor (10) being determined in advance for the particular water to be electrochemically activated which is used.

14. A method according to claim 13, **characterised in that** the dependency of the alkali metal chloride concentration on the specific electrical conductivity of the dilute water/alkali metal chloride solution added to the electrolysis reactor (10) is determined according to a calibration curve of the form
κₜₒₜₐₗ = κ_{w} + dκ/d[MeCl] · [MeCl]
kₜₒₜₐₗ being the specific electrical conductivity of the dilute water/alkali metal chloride solution added to the electrolysis reactor (10), k_{w} the specific electrical conductivity of the water to be disinfected which is used in each case, [MeCl] the alkali metal chloride concentration of the dilute water/alkali metal chloride solution and dκ/dMeCl] the water-specific gradient of the calibration curve.

15. A method according to any one of claims 1 to 14, **characterised in that** solely the electrochemically activated, dilute water/electrolyte solution produced in the anode compartment is used as the disinfectant.

## Revendications

1. Procédé de production d'un désinfectant par activation électrochimique (ECA) de l'eau, en ce que l'on ajoute à l'eau une solution électrolyte, notamment une solution de chlorure de sodium et/ou de potassium, et que l'eau chargée en solution électrolyte est chargée sous la forme d'une solution eau/électrolyte diluée dans un réacteur d'électrolyse (6) et alimentée en courant électrique par application d'une tension continue au niveau des électrodes (61, 62), avec au moins un espace cathodique doté d'une cathode (62) et avec au moins un espace anodique doté d'une anode (61) séparé dans l'espace de l'espace cathodique, notamment à l'aide d'un diaphragme ou d'une membrane, pour déplacer la solution eau/électrolyte diluée dans un état métastable adapté à la désinfection, la valeur pH de la solution eau/électrolyte diluée étant réglée dans l'espace anodique à une valeur située entre 2,7 et 3,5 et le potentiel d'oxydoréduction de la solution eau/électrolyte diluée dans l'espace anodique étant réglé à une valeur située entre 1240 mV et 1360 mV par rapport à l'électrode normale à l'hydrogène (SHE), en ce que :
(a) en cas de temps de séjour (T_{V}) préalablement prévu de la solution eau/électrolyte dans le réacteur d'électrolyse (6) et/ou en cas de débit-volume (V) préalablement prévu de la solution eau/électrolyte passant dans le réacteur d'électrolyse (6), un courant théorique statique (I_{soll, stat}) dépendant de la composition respective de l'eau à désinfecter est calculé entre les électrodes (61, 62) en fonction de la conductivité (κ) et/ou de la dureté (H) de l'eau selon une équation linéaire de la forme :
I_{soll, stat} = K₁ ˙κ + K₂
et/ou
I_{soll, stat} = K₃ · H + K₄
où K₁, K₂ ,K₃ et K₄ sont des constantes propres au réacteur ; et
(b) en présence d'un courant théorique statique préalablement prévu (I_{soll, stat}) entre les électrodes (61, 62) du réacteur d'électrolyse (6), un courant théorique dynamique (I_{soll, dyn}) dépendant du temps de séjour (T_{V}) respectif de la solution eau/électrolyte dans le réacteur d'électrolyse et/ou du débit-volume respectif de la solution eau/électrolyte traversant le réacteur d'électrolyse (6) est calculé en fonction d'une équation linéaire de la forme :
I_{soll, dyn} = K₅ · T_{V} + K₆
et/ou
I_{soll, dyn} = K₇ ˙ V' + K₈
où K₅, K₆, K₇ et K₈ sont des constantes propres au réacteur ; et
(c) un courant théorique total (I_{soll, ges}) est appliqué au niveau des électrodes (61, 62) du réacteur d'électrolyse (6) constitué par la somme du courant théorique statique (_{soll, stat}) et du courant théorique dynamique (I_{soll, dyn}):
I_{soll, ges} = I_{soll, stat} + I_{soll, dyn}.

2. Procédé selon la revendication 1, **caractérisé en ce que** la valeur pH de la solution eau/électrolyte diluée dans l'espace anodique est réglée à une valeur comprise entre 2,7 et 3,3, notamment à une valeur comprise entre 2,8 et 3,2.

3. Procédé selon la revendication 1 ou 2, **caractérisé en ce que** le potentiel d'oxydoréduction de la solution eau/électrolyte diluée dans l'espace anodique est réglé à une valeur comprise entre 1280 mV et 1360 mV par rapport à l'électrode SHE, notamment à une valeur comprise entre 1320 mV et 1360 mV par rapport à l'électrode SHE.

4. Procédé selon l'une quelconque des revendications 1 à 3, **caractérisé en ce que** la valeur pH de la solution eau/électrolyte diluée dans l'espace anodique est réglée en ajoutant la quantité correspondante de solution électrolyte.

5. Procédé selon l'une quelconque des revendications 1 à 4, **caractérisé en ce que** la valeur pH de la solution eau/électrolyte diluée dans l'espace anodique est réglée en commandant le temps de séjour (T_{V}) dans le réacteur d'électrolyse (6) et/ou le débit-volume (V') traversant ledit réacteur.

6. Procédé selon l'une quelconque des revendications 1 à 5, **caractérisé en ce que** pour régler la valeur pH de la solution eau/électrolyte diluée dans l'espace anodique du réacteur d'électrolyse (6) à une valeur pH comprise entre 2,7 et 3,5 en présence d'un courant théorique préalablement prévu entre les électrodes (61, 62) du réacteur d'électrolyse (6), un temps de séjour (T_{V}) dans le réacteur d'électrolyse (6) est calculé en fonction de la composition respective de l'eau à désinfecter et/ou un débit-volume (V') traversant ledit réacteur est calculé en fonction de la composition respective de l'eau de la solution eau/électrolyte diluée.

7. Procédé selon la revendication 6, **caractérisé en ce que** le temps de séjour (T_{V}) dans le réacteur d'électrolyse (6) et/ou le débit-volume (V') traversant ledit réacteur est calculé en fonction de la conductivité (κ) et/ou de la dureté (H) de l'eau.

8. Procédé selon la revendication 6 ou 7, **caractérisé en ce que** le temps de séjour (T_{V}) et/ou le débit-volume (V') est calculé en fonction de l'équation linéaire de la forme :
T_{V} = k₁ ˙ κ + k₂
et/ou
V' = k₃ ˙ H + k₄
où k₁, k₂ ,k₃ et k₄ sont des constantes propres au réacteur.

9. Procédé selon l'une quelconque des revendications 6 à 8, **caractérisé en ce que** la quantité de solution électrolyte ajoutée est maintenue pour l'essentiel à un niveau constant.

10. Procédé selon l'une quelconque des revendications 1 à 9, **caractérisé en ce que** la concentration d'électrolyte de la solution eau/électrolyte diluée ajoutée au réacteur d'électrolyse (10) est réglée à une valeur de 20 g/l maximum.

11. Procédé selon l'une quelconque des revendications 1 à 10, **caractérisé en ce que** la conductivité électrique spécifique de l'eau à activer électrochimiquement est réglée avant l'ajout de la solution électrolyte à une valeur maximale de 350 µS/cm.

12. Procédé selon l'une quelconque des revendications 1 à 11, **caractérisé en ce que** le réglage de la concentration d'électrolyte, notamment de la concentration en chlorure alcalin, de la solution eau/électrolyte diluée ajoutée au réacteur d'électrolyse (10), notamment de la solution eau/chlorure alcalin diluée, est effectué par réglage de la quantité d'eau à activer électrochimiquement ajoutée à la solution électrolyte.

13. Procédé selon la revendication 12, **caractérisé en ce que** le réglage de la concentration en chlorure alcalin de la solution eau/chlorure alcalin diluée ajoutée au réacteur d'électrolyse (10) est réalisé en fonction de la conductivité électrique spécifique correspondante de la solution eau/chlorure alcalin ajoutée au réacteur d'électrolyse (10), l'interdépendance entre la concentration en chlorure alcalin de la conductivité électrique spécifique de la solution eau/chlorure alcalin diluée ajoutée au réacteur d'électrolyse (10) étant calculée au préalable pour l'eau à activer électrochimiquement respectivement ajoutée.

14. Procédé selon la revendication 13, **caractérisé en ce que** l'interdépendance de la concentration en chlorure alcalin et de la conductivité électrique spécifique de la solution eau/chlorure alcalin diluée ajoutée au réacteur d'électrolyse (10) est fonction d'une droite d'étalonnage de la forme :
κ_{ges} = κ_{w} + dκ/d[MeCl] ˙ [MeCl]
où κ_{ges} est la conductivité électrique spécifique de la solution eau/chlorure alcalin diluée ajoutée au réacteur d'électrolyse (10), κ_{w} est la conductivité électrique spécifique de l'eau à désinfecter respectivement ajoutée, [MeCl] est la concentration en chlorure alcalin de la solution eau/chlorure alcalin diluée et dκ/d[MeCl] est la pente spécifique de la droite d'étalonnage.

15. Procédé selon l'une quelconque des revendications 1 à 14, **caractérisé en ce que** seule la solution eau/électrolyte diluée produite dans l'espace anodique et activée électrochimiquement sert de désinfectant.
